(19) Europäisches Patentamt
European Patent Office
Office européen des brevets

(11) **EP 4 208 216 B1**

(12) **EUROPÄISCHE PATENTSCHRIFT**

(45) Veröffentlichungstag und Bekanntmachung des Hinweises auf die Patenterteilung:
**09.04.2025 Patentblatt 2025/15**

(21) Anmeldenummer: **21769911.5**

(22) Anmeldetag: **24.08.2021**

(51) Internationale Patentklassifikation (IPC):
*A61M 1/36* *(2006.01)* *A61M 1/16* *(2006.01)*

(52) Gemeinsame Patentklassifikation (CPC):
**A61M 1/1682; A61M 1/3647; A61M 1/365;**
A61M 2205/3331

(86) Internationale Anmeldenummer:
**PCT/EP2021/073402**

(87) Internationale Veröffentlichungsnummer:
**WO 2022/048955 (10.03.2022 Gazette 2022/10)**

(54) **AUTOMATISCHES PRIMEN UND SPÜLEN EINER EXTRAKORPORALEN BLUTBEHANDLUNGSVORRICHTUNG**

AUTOMATIC PRIMING AND RINSING OF AN EXTRACORPOREAL BLOOD TREATMENT APPARATUS

AMORÇAGE ET RINÇAGE AUTOMATIQUES D'UN APPAREIL DE TRAITEMENT SANGUIN EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.09.2020 DE 102020122937**

(43) Veröffentlichungstag der Anmeldung:
**12.07.2023 Patentblatt 2023/28**

(73) Patentinhaber: **B. Braun Avitum AG**
**34212 Melsungen (DE)**

(72) Erfinder: **WAGNER, André**
**34117 Kassel (DE)**

(74) Vertreter: **Winter, Brandl - Partnerschaft mbB**
**Alois-Steinecker-Straße 22**
**85354 Freising (DE)**

(56) Entgegenhaltungen:
**WO-A1-2013/183599      JP-A- 2001 245 970**
**JP-A- 2013 248 335**

**Beschreibung**

Die vorliegende Offenbarung betrifft eine extrakorporale Blutbehandlungsvorrichtung, insbesondere Dialysemaschine, welche zum automatischen Primen und Spülen derselben vorbereitet bzw. eingerichtet ist. Weiterhin betrifft die vorliegende Offenbarung ein Verfahren zum automatischen Primen und Spülen einer extrakorporalen Blutbehandlungsvorrichtung.

Technischer Hintergrund

Aus der Praxis sind extrakorporale (Blut-) Kreisläufe zum Führen von Blut außerhalb eines Patientenkörpers während eines Blutreinigungsverfahrens bekannt. Derartige extrakorporale Kreisläufe werden vor Beginn einer Behandlung mit einer Priminglösung bzw. Primingflüssigkeit befüllt. Dieses Befüllen des extrakorporalen Kreislaufs und eines Dialysators mit Priminglösung bzw. Primingflüssigkeit (Primen) dient einer Vorbereitung des extrakorporalen Kreislaufs und des Dialysators für die nachfolgende Behandlung. Insbesondere dient das Primen dazu, im extrakorporalen Kreislauf und im Dialysator vor der Behandlung noch vorhandene Luft durch eine physiologisch verträgliche Flüssigkeit oder Lösung zu verdrängen, um ein Einbringen dieser Luft in das Gefäßsystem des Patienten während und insbesondere zu Beginn der Blutbehandlung zu verhindern.

Neben dem Primen bzw. Primingvorgang wird zur Vorbereitung des extrakorporalen Kreislaufs und des Dialysators für die nachfolgende Behandlung außerdem üblicherweise ein Spülen bzw. Spülvorgang des extrakorporalen Kreislaufs und des Dialysators vorgenommen. Das Spülen bzw. der Spülvorgang wird gewöhnlich gleichzeitig oder (unmittelbar) nachfolgend an das Primen bzw. den Primingvorgang durchgeführt. Das Spülen bzw. der Spülvorgang dient insbesondere dazu, dass Reststoffe/ Pyrogene aus dem extrakorporalen Kreislauf und aus dem Dialysator, insbesondere aus einer Membran desselben (die Pyrogene sind membrangängig), herausgespült werden. Derartige Reststoffe/ Pyrogene können einem Produktions-, Verpackungs- oder Aufrüstprozess entstammen und stellen eine potentielle Gefährdung des Patienten dar.

Wenn der Dialysator und ein den extrakorporalen (Blut-)Kreislauf bildendes Schlauchsystem vorbereitet sind, das heißt wenn das Primen und Spülen derselben durchgeführt wurde, kann der Patient grundsätzlich angelegt werden, und die Priming- bzw. Spülflüssigkeit wird aus dem vollständig gefüllten System abgelassen und auf einer Seite durch das Blut des Patienten ersetzt.

Die vorliegende Offenbarung betrifft die Vorbereitung der extrakorporalen Blutbehandlungsvorrichtung, also das Primen und Spülen. Ein Anlegen/ eine Behandlung eines Patienten ist in anderen Worten nicht Gegenstand der vorliegenden Offenbarung, so dass offenbarungsgemäß kein Kontakt der extrakorporalen Blutbehandlungsvorrichtung mit einem Patienten vorgesehen ist.

Stand der Technik

Aus dem Stand der Technik ist ein Primen/ Priming über einen externen Kochsalzbeutel bekannt. Dabei wird ein Kochsalzbeutel mit einem Schlauch des extrakorporalen Kreislaufs (mit einer Seite des Blutschlauchsystems) verbunden und die Primingflüssigkeit wird in das Schlauchsystem und den Dialysator gefüllt. Damit der Dialysator geeignet mit Flüssigkeit gefüllt wird, muss dieser zwischendurch gedreht werden, um noch vorhandene Luftblasen aus dem System zu entfernen. Die verbrauchte Flüssigkeit wird dabei in einem leeren Beutel aufgefangen.

Ferner ist aus dem Stand der Technik das sogenannte Online-Priming bekannt. Beim Online-Priming wird Dialysierflüssigkeit von der Blutbehandlungsvorrichtung bereitgestellt und über einen Substituatanschluss mit Hilfe einer arteriellen Blutpumpe in das Blutschlauchsystem und den Dialysator gegeben. Auch bei diesem Priming-Verfahren muss der Dialysator gedreht werden, um noch vorhandene Luftblasen aus dem System zu entfernen.

Diese beiden genannten Priming-Verfahren haben den Nachteil, dass grundsätzlich manuell vorzunehmende Schritte von einem Anwender erforderlich sind, beispielsweise beim Drehen des Dialysators. Aufgrund eines hohen Kostendrucks und zum Teil strengen Richtlinien (z.B. des Centers for Disease Control and Preventions in den Vereinigten Staaten) ist grundsätzlich eine automatische Vorbereitung der extrakorporalen Blutbehandlungsvorrichtung (Primen und Spülen) wünschenswert. Dabei sollen bevorzugt kein Kochsalzlösungsbeutel und keine Einwegartikel, welche vor der Therapie entfernt und entsorgt werden müssen, zum Einsatz kommen.

Aus dem Stand der Technik ist grundsätzlich auch bekannt, dass zum Primen Dialysierflüssigkeit/ Dialysat (anstelle einer Kochsalzlösung) verwendet wird. Dabei wird die Dialysierflüssigkeit über eine Membran des Dialysators in das Blutschlauchsystem befördert. Wenn dabei die arterielle und venöse Blutschlauchleitung kurzgeschlossen werden, kann von der Dialysierflüssigkeitsseite aus mittels einer geeigneten Pumpenbetätigung und mittels geeigneten Ventilstellungen Dialysierflüssigkeit in den Dialysator und das Blutschlauchsystem gepumpt werden und diese somit befüllt werden. In diesem Zusammenhang wird beispielsweise auf die EP 3 127 564 B1 oder die EP 1 457 218 A1 verwiesen.

Weitere automatische Priming-Verfahren sind beispielsweise aus der US 9,579,440 B2 oder der EP 2 361 643 B1 bekannt.

Die WO 2013/183599 A1 offenbart eine extrakorporale Blutbehandlungsvorrichtung gemäß dem Oberbegriff des Anspruchs 1.

Der Stand der Technik hat insbesondere den Nachteil, dass keine geeigneten automatischen Spülprozesse existieren, welche den extrakorporalen Kreislauf und den Dialysator in zufriedenstellender Weise (zum Entfernen der Reststoffe/ Pyrogene) durchspülen können.

Kurzbeschreibung der Offenbarung

Aufgabe der vorliegenden Offenbarung ist es, ein verbessertes automatisches Vorbereiten (umfassend sowohl Primen als auch Spülen) einer extrakorporalen Blutbehandlungsvorrichtung bereitzustellen. Insbesondere soll sowohl beim Primen als auch beim Spülen des Dialysators und des extrakorporalen Schlauchsystems eine Anzahl von erforderlichen Einwegartikeln/ Einmalartikeln reduziert werden bzw. sollen bevorzugt wenige/ keine zusätzlichen Einwegartikel/ Einmalartikel und kein Substitutionsanschluss erforderlich sein.

Diese Aufgabe wird durch eine extrakorporale Blutbehandlungsvorrichtung nach Anspruch 1 und ein Verfahren zum automatischen Primen und Spülen einer extrakorporalen Blutbehandlungsvorrichtung nach Anspruch 6 gelöst. Vorteilhafte Ausführungsformen bzw. Weiterbildungen sind Gegenstand der Unteransprüche und/oder werden nachfolgend erläutert.

Die vorliegende Offenbarung betrifft zunächst eine extrakorporale Blutbehandlungsvorrichtung mit: einem extrakorporalen Kreislauf umfassend einen arteriellen Abschnitt und einen venösen Abschnitt, einem Dialysator und einem Dialysierflüssigkeitskreislauf, wobei: der extrakorporale Kreislauf und der Dialysierflüssigkeitskreislauf über eine in dem Dialysator vorgesehene Membran voneinander getrennt sind; die extrakorporale Blutbehandlungsvorrichtung zum Primen und Spülen des extrakorporalen Kreislaufs und des Dialysators vor einem erstmaligen Verwenden des extrakorporalen Kreislaufs und des Dialysators vorbereitet bzw. eingerichtet ist und dazu der arterielle Abschnitt und der venöse Abschnitt über eine Verbindervorrichtung kurzgeschlossen bzw. verbunden sind; der Dialysierflüssigkeitskreislauf ein Dialysatoreinlassventil, ein Dialysatorauslassventil und zumindest eine Flusspumpe aufweist; der extrakorporale Kreislauf zumindest ein Entlüftungsventil aufweist; und die extrakorporale Blutbehandlungsvorrichtung ferner eine Steuereinheit enthält, welche eingerichtet ist, das Primen derart zu steuern, dass eine Flüssigkeit, insbesondere Priming-/ Spül-/ Dialysierflüssigkeit, von dem Dialysierflüssigkeitskreislauf über die Membran des Dialysators an den extrakorporalen Blutkreislauf geliefert wird. Die Steuereinheit ist ferner eingerichtet, das gleichzeitig mit dem Primen oder unmittelbar nachfolgend auf das Primen durchzuführende Spülen der extrakorporalen Blutbehandlungsvorrichtung, insbesondere des Dialysators und des extrakorporalen Kreislaufs, derart zu steuern, dass zum Spülen ein Druckunterschied zwischen dem extrakorporalen Kreislauf und dem Dialysierflüssigkeitskreislauf erzeugt und ein Flüssigkeitstransfer über die Membran des Dialysators, insbesondere von dem extrakorporalen Kreislauf zu dem Dialysierflüssigkeitskreislauf, bewirkt wird.

In anderen Worten stellt die vorliegende Offenbarung eine extrakorporale Blutbehandlungsvorrichtung bereit, welche es ermöglicht, dass sowohl das Primen als auch das Spülen realisiert werden, indem eine Flüssigkeit (Priming-/ Spül-/ Dialysierflüssigkeit) über die Membran des Dialysators von der Dialysierflüssigkeitsseite geliefert wird. Es wird somit offenbarungsgemäß kein separater Beutel mit Kochsalzlösung/ Priming-/ Spülflüssigkeit bzw. kein Substituatanschluss an der Maschine zum Primen und zum Spülen benötigt. Das automatische Primen und Spülen der vorliegenden Offenbarung ist demnach auch auf extrakorporale Blutbehandlungsmaschinen/ Dialysemaschinen anwendbar, welche keinen Substituatanschluss/ -port besitzen. Da kein externer Kochsalzbeutel verwendet wird, ergibt sich durch das offenbarungsgemäße automatische Primen und Spülen ferner eine Zeitersparnis für das Pflegepersonal. Das offenbarungsgemäße automatische Primen und Spülen ermöglicht es ferner, dass ein Befüllen und Spülen des Dialysators möglich ist, ohne denselben drehen zu müssen, so dass auch dieser Arbeitsschritt des Pflegepersonals entfällt. Denn der Dialysator wird offenbarungsgemäß bevorzugt von unten gefüllt/ befüllt und möglicherweise in dem Dialysator verbleibende Luftblasen werden bevorzugt durch den offenbarungsgemäßen Spülprozess/ -vorgang entfernt.

Um das offenbarungsgemäße automatische Primen und Spülen zu ermöglichen, sind der arterielle Abschnitt und der venöse Abschnitt des extrakorporalen Kreislaufs über eine Verbindervorrichtung kurzgeschlossen bzw. verbunden.

Die Verbindervorrichtung ist bevorzugt ein Adapter, in welchen sowohl ein Ende des arteriellen Abschnitts als auch ein Ende des venösen Abschnitts eingesteckt und diese dadurch fluidisch verbunden werden können. Da bevorzugt sowohl an dem Ende des arteriellen Abschnitts als auch an dem Ende des venösen Abschnitts ein Luer-Anschluss vorgesehen ist, ist der Adapter in vorteilhafter Weise zum Verbinden/ Anschließen von zwei Luer-Anschlüssen eingerichtet. Beispielsweise ist der Combifix®-Adapter der Anmelderin der vorliegenden Patentanmeldung eine geeignete Verbindervorrichtung. Die offenbarungsgemäße Verbindervorrichtung ist jedoch nicht darauf beschränkt. Grundsätzlich ist jeder beliebige Verbinder/ jedes beliebige Verbinderstück denkbar, solange er/ es ermöglicht, dass in geeigneter Weise der arterielle Abschnitt des extrakorporalen Kreislaufs mit dem venösen Abschnitt des extrakorporalen Blutkreislaufs verbunden/ kurzgeschlossen werden kann.

Das automatische Primen und/oder Spülen der vorliegenden Offenbarung wird bevorzugt durch eine (geeignete) Steuerung der Ventilstellungen (OFFEN/ GESCHLOSSEN) der in der extrakorporalen Blutbehandlungsvorrichtung vorhandenen/ vorgesehenen Ventile realisiert.

Weiter bevorzugt wird das automatische Primen und/oder Spülen der vorliegenden Offenbarung (zusätzlich auch) durch eine (geeignete) Steuerung der Flusspumpe, welche in dem Dialysierflüssigkeitskreislauf vorhanden/ vorgesehen ist, realisiert.

Offenbarungsgemäß sind in dem Dialysierflüssigkeitskreislauf sowohl ein Dialysatoreinlassventil als auch ein Dialysatorauslassventil vorgesehen. Das Dialysatoreinlassventil ist bevorzugt an einem Dialysierflüssigkeitszufluss stromaufwärts des Dialysators vorgesehen/ angeordnet. Das Dialysatorauslassventil ist bevorzugt an einem Dialysierflüssigkeitsabfluss stromabwärts des Dialysators vorgesehen/ angeordnet.

In vorteilhafter Weise ist in dem Dialysierflüssigkeitszufluss stromaufwärts des Dialysatoreinlassventils eine Flusspumpe-Eingang vorgesehen/ angeordnet. Weiter bevorzugt ist in dem Dialysierflüssigkeitsabfluss stromabwärts des Dialysatorauslassventils eine Flusspumpe-Ausgang vorgesehen/ angeordnet. Es kann offenbarungsgemäß jedoch auch lediglich eine Flusspumpe, also entweder die Flusspumpe-Eingang oder die Flusspumpe-Ausgang vorgesehen sein. Die Flusspumpe-Eingang und/oder die Flusspumpe-Ausgang ist/ sind bevorzugt als Zahnradpumpe/n ausgebildet. Die zumindest eine Flusspumpe ist bevorzugt eingerichtet, Priming-/ Spül-/ Dialysierflüssigkeit von dem Dialysierflüssigkeitskreislauf über die Membran des Dialysators an den extrakorporalen Kreislauf zu liefern.

Der extrakorporale Kreislauf weist bevorzugt zumindest einen Drucksensor auf, welcher einen Druck in dem extrakorporalen Kreislauf misst/ überwacht und Informationen über einen Druckverlauf in dem extrakorporalen Kreislauf der Steuereinheit zur Verfügung stellt. Die Steuereinheit der vorliegenden Offenbarung ist bevorzugt als ein Prozessor, insbesondere als eine zentrale Rechen-/ bzw. Verarbeitungseinheit (CPU), ausgebildet. Beispielsweise ist der zumindest eine Drucksensor ein arterieller Drucksensor in dem arteriellen Abschnitt des extrakorporalen Kreislaufs stromaufwärts einer Blutpumpe (bevorzugt als Rollenpumpe ausgebildet, um durch Quetschen eines Schlauchs ein Fluid/ eine Flüssigkeit zu fördern), oder ein Dialysatoreingangsdrucksensor in dem arteriellen Abschnitt stromaufwärts des Dialysators, oder ein venöser Drucksensor in dem venösen Abschnitt des extrakorporalen Kreislaufs. Bevorzugt sind in dem extrakorporalen Kreislauf sowohl der arterielle Drucksensor als auch der Dialysatoreingangsdrucksensor als auch der venöse Drucksensor vorgesehen.

In dem extrakorporalen Kreislauf ist bevorzugt zumindest eine Expansionskammer vorgesehen, welche insbesondere als eine Luftfalle ausgebildet ist. Beispielsweise ist in dem venösen Abschnitt des extrakorporalen Kreislaufs eine venöse Expansionskammer bzw. eine venöse Luftfalle vorgesehen. Zusätzlich oder alternativ kann in dem arteriellen Abschnitt des extrakorporalen Kreislaufs eine arterielle Expansionskammer bzw. eine arterielle Luftfalle vorgesehen sein.

Der zumindest eine Drucksensor, welcher in dem extrakorporalen Kreislauf vorgesehen sein kann, kann den Druck in dem extrakorporalen Kreislauf an der zumindest einen Expansionskammer/ Luftfalle messen/ abnehmen/ überwachen. Beispielsweise kann, wenn eine venöse Expansionskammer bzw. Luftfalle vorgesehen ist, an dieser der venöse Druck über einen venösen Drucksensor abgenommen/ gemessen werden. Alternativ oder zusätzlich kann, wenn eine arterielle Expansionskammer bzw. Luftfalle vorgesehen ist, an dieser der arterielle Druck oder der Dialysatoreingangsdruck über einen arteriellen Drucksensor oder einen Dialysatoreingangsdrucksensor abgenommen/ gemessen werden.

Hinter dem zumindest einen Drucksensor (z.B. arterieller Drucksensor, venöser Drucksensor, Dialysatoreingangsdrucksensor) ist offenbarungsgemäß bevorzugt eine Pegel- oder Levelregulierungspumpe vorgesehen/ angeordnet, welche insbesondere als ein Luftkompressor ausgebildet ist. Die Pegel- oder Levelregulierungspumpe dient insbesondere einem Einstellen des Pegels/ Levels der Expansionskammer/ Luftfalle und kann Luft aus dem Schlauchsystem/ dem extrakorporalen Kreislauf entfernen oder Luft in das Schlauchsystem/ den extrakorporalen Kreislauf drücken. Beispielsweise kann eine venöse Expansionskammer/ Luftfalle vorgesehen sein, an welcher der venöse Druck über den venösen Drucksensor abgenommen/ gemessen wird, und kann hinter dem venösen Drucksensor die Pegel- oder Levelregulierungspumpe vorgesehen sein.

Offenbarungsgemäß weist der extrakorporale Kreislauf zumindest ein Entlüftungsventil auf. In anderen Worten soll offenbarungsgemäß irgendwo (an einer beliebigen Stelle/ Position) in dem extrakorporalen Kreislauf zumindest ein Entlüftungsventil vorgesehen sein, über welches Luft aus dem extrakorporalen Kreislauf entweichen/ freigegeben werden kann (über ein geöffnetes Ventil).

Bevorzugt ist das zumindest eine Entlüftungsventil an einem Abschnitt (Schlauchabschnitt) des extrakorporalen Kreislaufs vorgesehen, über welchen Luft aus einer vorgesehenen Expansionskammer/ Luftfalle entweichen kann. Beispielsweise kann das Entlüftungsventil hinter einem Drucksensor, welcher den Druck an der Expansionskammer/ Luftfalle misst/ abnimmt, besonders bevorzugt hinter der Pegel-oder Levelregulierungspumpe angeordnet/ vorgesehen sein.

Gemäß einem bevorzugten Ausführungsbeispiel der vorliegenden Offenbarung ist bevorzugt eine venöse Expansionskammer/ Luftfalle vorgesehen, an welcher über einen venösen Drucksensor der venöse Druck gemessen/ abgenommen wird, und kann Luft aus der venösen Expansionskammer/ Luftfalle über einen Abschnitt (Schlauchabschnitt) des extrakorporalen Kreislaufs entweichen, und ist in diesem Abschnitt des extrakorporalen Kreislaufs die Pegel- oder Levelregulierungspumpe und das Entlüftungsventil vorgesehen/ angeordnet. Alternativ oder zusätzlich kann auch eine arterielle Expansionskammer/ Luftfalle vorgesehen sein, an welcher über einen Drucksensor der Druck gemessen/ abgenommen wird, und kann Luft aus der arteriellen Expansionskammer/ Luftfalle über einen Abschnitt (Schlauchabschnitt) des extrakorporalen Kreislaufs entweichen, und ist in diesem Abschnitt des extrakorporalen Kreislaufs die Pegel- oder Levelregulierungspumpe und das Entlüftungsventil vorgesehen/ angeordnet.

Offenbarungsgemäß ist die Steuereinheit der extrakorporalen Blutbehandlungsvorrichtung eingerichtet, das Primen derart zu steuern, dass eine Flüssigkeit, insbesondere Priming-/ Spül-/ Dialysierflüssigkeit, von dem Dialysierflüssigkeitskreislauf über die Membran des Dialysators an den extrakorporalen Blutkreislauf geliefert wird.

In vorteilhafter Weise ist die Steuereinheit dabei eingerichtet, zum Steuern des Primens das Dialysatoreinlassventil und das zumindest eine Entlüftungsventil zu öffnen, und das Dialysatorauslassventil zu schließen, und die zumindest eine Flusspumpe, insbesondere Flusspumpe-Eingang (stromaufwärts des Dialysators im Dialysierflüssigkeitskreislauf), anzusteuern, damit diese die Flüssigkeit, insbesondere Priming-/ Spül-/ Dialysierflüssigkeit, mit einem erforderlichen Druck in den Dialysator pumpt. Es versteht sich dabei von selbst, dass, wenn mehr als ein Entlüftungsventil in dem extrakorporalen Kreislauf vorgesehen ist, jedes Entlüftungsventil bevorzugt geöffnet ist, damit Luft beim Primen/ Füllen des extrakorporalen Kreislaufs mit Priming- bzw. Spülflüssigkeit über das Entlüftungsventil bzw. die Entlüftungsventile entweichen kann.

Der von der Flusspumpe bereitgestellte erforderliche Druck kann in Abhängigkeit des verwendeten Dialysators, insbesondere in Abhängigkeit des Ultrafiltrationskoeffizienten und des Volumens des Dialysators, und in Abhängigkeit des verwendeten extrakorporalen Kreislaufs/ Schlauchsystems, insbesondere in Abhängigkeit des Volumens des extrakorporalen Kreislaufs, bestimmt werden. Insbesondere kann der von der Flusspumpe aufzubringende erforderliche Druck in Abhängigkeit von dem verwendeten Dialysator und dem verwendeten extrakorporalen Kreislauf/ A/V-Schlauchsystem wie folgt berechnet werden (siehe nachfolgende Gleichung 1)

$$\Delta p = \frac{V_{AV} + V_{Dialysator}}{(\frac{K_{UF}}{60\ min})}$$

(Gleichung 1)

In anderen Worten werden gemäß Gleichung 1, um den von der Flusspumpe aufzubringenden erforderlichen Druck $\Delta p$ zu bestimmen, zunächst die Volumina des extrakorporalen Kreislaufs/ Schlauchsystems $V_{AV}$ und des Dialysators $V_{Dialysator}$ addiert, und die Summe durch einen Ultrafiltrationskoeffizienten geteilt, welcher die minütliche Ultrafiltration in ml angibt, die pro mmHg Transmembrandruck (TMP) erzielt wird. Somit kann durch obige Gleichung 1 der von der Dialysierflüssigkeitsseite erforderliche Druck, welcher von der Flusspumpe bereitgestellt wird, berechnet werden, mittels welchem der extrakorporale Kreislauf/ das extrakorporale Schlauchsystem und der Dialysator in einer Minute befüllt werden können, das heißt in anderen Worten das Primen/ Priming eine Minute dauert.

Um das gesamte Volumen über die (Hohlfaser-)Membran des Dialysators strömen/ fließen zu lassen, wird in vorteilhafter Weise nicht nur von der Dialysierflüssigkeitsseite die Priming- bzw. Spülflüssigkeit durch die Membran des Dialysators gedrückt, sondern wird auch auf der Seite des extrakorporalen Kreislaufs in geeigneter Weise gezogen, insbesondere indem ein negativer Druck bzw. Unterdruck in dem extrakorporalen Kreislauf erzeugt wird (beispielsweise mittels der Blutpumpe oder mittels der Pegel-oder Levelregulierungspumpe). Dabei hat es sich als vorteilhaft herausgestellt, wenn der negative Wert des Drucks verwendet wird, mittels welchem auf der Dialysierflüssigkeitsseite die Priming- bzw. Spülflüssigkeit durch die Membran des Dialysators gedrückt wird.

Um das Primen/ Priming offenbarungsgemäß durchführen zu können, kann in vorteilhafter Weise der Druck in dem extrakorporalen Kreislauf/ Schlauchsystem abgebaut werden, wenn die Priming- bzw. Spülflüssigkeit über die Membran des Dialysators in den extrakorporalen Kreislauf/ das extrakorporale Schlauchsystem gedrückt bzw. gezogen wird. Dies wird offenbarungsgemäß durch das zumindest eine Entlüftungsventil ermöglicht, über welches Luft aus dem extrakorporalen Kreislauf entweichen/ ausströmen kann.

Gemäß einer bevorzugten Ausführungsform wird das Primen/ Priming zeitgesteuert durchgeführt. Insbesondere ist ein zeitabhängiges Primen/ Priming denkbar, welches in Abhängigkeit von dem verwendeten Dialysator (Volumen und $K_{UF}$), dem verwendeten extrakorporalen Kreislauf (Volumen) und dem anliegenden Druck durchgeführt wird. Alternativ kann das Primen/ Priming auch volumengesteuert durchgeführt werden. Insbesondere ist es dabei auch denkbar, die Pegel- oder Levelregulierungspumpe zu verwenden und mit dieser das Luftvolumen während der Entlüftung zu messen. Bevorzugt kann das Primen/ Priming dann so gesteuert werden, dass es beendet wird, wenn das Volumen des Systems erreicht ist.

Bevorzugt ist die Steuereinheit eingerichtet, nach Abschluss des Primens und vor Beginn des Spülens das Dialysatoreinlassventil zu schließen, die zumindest eine Flusspumpe, insbesondere Flusspumpe-Eingang, zu stoppen, das zumindest eine Entlüftungsventil geöffnet zu halten, und das Dialysatorauslassventil geschlossen zu halten.

Das Spülen der extrakorporalen Blutbehandlungsvorrichtung, insbesondere des Dialysators und des extrakorporalen Kreislaufs, wird offenbarungsgemäß derart gesteuert, dass zum Spülen ein Druckunterschied zwischen dem extrakorporalen Kreislauf und dem Dialysierflüssigkeitskreislauf erzeugt wird, zum Bewirken eines Transfers der Flüssigkeit über die Membran des Dialysators von dem extrakorporalen Kreislauf zu dem Dialysierflüssigkeitskreislauf.

In vorteilhafter Weise wird der Druckunterschied zwischen dem extrakorporalen Kreislauf und dem Dialysierflüssigkeitskreislauf erzeugt, indem zunächst der Druck in dem extrakorporalen Kreislauf über einen Fluidfluss/ Flüssigkeitsfluss von Priming-/ Spül-/ Dialysierflüssigkeit von dem Dialysierflüssigkeitskreislauf über die Membran des Dialysators in den extrakorporalen Kreislauf (bei geschlossenem/n (Entlüftungs)Ventil/en in dem extrakorporalen Kreislauf) aufgebaut/ erhöht wird, und anschließend der Druck in dem Dialysierflüssigkeitskreislauf (beispielsweise durch Öffnen eines Ventils, insbesondere des Dialysatorauslassventils) abgelassen/ entfernt wird. Dieser so entstehende Druckunterschied ist dann zunächst in dem extrakorporalen Kreislauf gespeichert, beispielsweise in einer Expansionskammer/ Ausgleichskammer/ Luftfalle. Der gespeicherte Druckunterschied (Überdruck) in dem extrakorporalen Kreislauf wird offenbarungsgemäß bevorzugt über einen Fluidfluss von dem extrakorporalen Kreislauf über die Membran des Dialysators hin/ zurück zu dem Dialysierflüssigkeitskreislauf abgebaut. Dazu ist in vorteilhafter Weise das Ventil, insbesondere das Dialysatorauslassventil, geöffnet. Der offenbarungsgemäße Spülvorgang wird somit in anderen Worten insbesondere dadurch realisiert, dass Priming- bzw. Spülflüssigkeit zunächst von dem Dialysierflüssigkeitskreislauf in den extrakorporalen Kreislauf fließt, und danach wieder von dem extrakorporalen Kreislauf zurück in den Dialysierflüssigkeitskreislauf fließt.

Insgesamt wird offenbarungsgemäß somit bevorzugt der extrakorporale Kreislauf/ das AV-Schlauchsystem in einem ersten Schritt auf einen maximalen Druck befüllt. Danach folgt bevorzugt ein Druckablass durch Öffnen des Dialysatorauslassventils. Dies hat zur Folge, dass auch der Druck in dem extrakorporalen Kreislauf relaxieren kann, indem die gestaute Flüssigkeit bevorzugt in den Dialysierflüssigkeitskreislauf zurückfließt und über das Dialysatorauslassventil abgelassen wird. In anderen Worten wird das in der Expansionskammer/ Ausgleichskammer/ Luftfalle gespeicherte Volumen reduziert, indem das Flüssigkeitsvolumen über die (Hohlfaser-) Membran des Dialysators gedrückt/ gepresst wird.

In vorteilhafter Weise wird der beschriebene Spülvorgang/ das Spülen mehrfach durchgeführt bzw. zyklisch wiederholt, um möglichst alle sich in dem extrakorporalen Kreislauf und/oder dem Dialysator befindlichen Reststoffe/ Pyrogene zu entfernen. Dabei kann die Dauer des gesamten Spülprozesses bevorzugt individuell eingestellt werden. Die Anzahl der Wiederholungen des Spülvorgangs/ des Spülens hängt insbesondere von dem gewünschten bzw. eingestellten bzw. erforderlichen Spülvolumen ab. Beispielsweise kann für einen extrakorporalen Kreislauf/ ein A/V-Schlauchsystem ein minimales Spülvolumen vorgegeben sein. Für typische/ handelsübliche extrakorporale Kreisläufe hat sich dabei insbesondere ergeben, dass bevorzugt 5 bis 30, besonders bevorzugt 10 bis 25, Wiederholungen vorgenommen werden müssen, um das Spülen geeignet durchzuführen. Insbesondere hat sich dabei auch herausgestellt, dass pro Spülvorgang etwa ein Spülvolumen von 20 ml bis 40 ml, bevorzugt von 25 ml bis 35 ml, besonders bevorzugt etwa 30 ml, erreicht wird. Es gilt jedoch selbstverständlich, dass das Spülvolumen pro Spülvorgang, sowie die Anzahl der Wiederholungen insbesondere davon abhängen, wie viel Flüssigkeit beim Druckaufbau zusätzlich in den extrakorporalen Kreislauf gegeben werden kann. Das heißt, das Spülvolumen pro Spülvorgang und die Anzahl der Wiederholungen sind in großem Maße von dem verwendeten extrakorporalen Kreislauf/ A/V-Schlauchsystem, dem verwendeten Dialysator, etc. abhängig.

Bevorzugt ist die Steuereinheit eingerichtet, zum Steuern des Spülens: den Druckunterschied zwischen dem extrakorporalen Kreislauf und dem Dialysierflüssigkeitskreislauf derart zu erzeugen, dass zunächst ein vorbestimmter maximaler Druck der Flüssigkeit, insbesondere Priming-/ Spül-/ Dialysierflüssigkeit, in dem Dialysierflüssigkeitskreislauf und dem extrakorporalen Kreislauf über ein in dem Dialysierflüssigkeitskreislauf vorgesehenes Druckerzeugungsmittel, insbesondere die zumindest eine Flusspumpe, erzeugt wird, und dann der vorbestimmte maximale Druck in dem Dialysierflüssigkeitskreislauf abgebaut wird. **In** anderen Worten erfolgt bevorzugt eine Druckrelaxation in dem Dialysierflüssigkeitskreislauf, beispielsweise indem ein in dem Dialysierflüssigkeitskreislauf vorgesehenes Ventil (bevorzugt das Dialysatorauslassventil) geöffnet wird. Dabei wird in vorteilhafter Weise der vorbestimmte maximale Druck in dem extrakorporalen Kreislauf über den Flüssigkeitstransfer von dem extrakorporalen Kreislauf zu dem Dialysierflüssigkeitskreislauf über die Membran des Dialysators abgebaut.

Das Spülen wird offenbarungsgemäß somit bevorzugt durch einen gespeicherten Druckunterschied in dem extrakorporalen Kreislauf und eine Steuerung/ Ansteuerung/ Betätigung der in der extrakorporalen Blutbehandlungsvorrichtung vorhandenen Ventile (Dialysatoreinlassventil, Dialysatorauslassventil, Entlüftungsventil, etc.), Pumpen, etc. realisiert. Dabei hat sich insbesondere die nachfolgende Steuerung als vorteilhaft herausgestellt:
**In** vorteilhafter Weise ist die Steuereinheit eingerichtet, zum Steuern des Spülens: das Dialysatoreinlassventil zu öffnen oder geöffnet zu halten, das zumindest eine Entlüftungsventil zu schließen oder geschlossen zu halten, das Dialysatorauslassventil zu schließen oder geschlossen zu halten; und die zumindest eine Flusspumpe anzusteuern, damit diese die Flüssigkeit, insbesondere Priming-/ Spül-/ Dialysierflüssigkeit mit einem (vorbestimmten) erforderlichen Druck in den Dialysator pumpt, und zwar bis ein vorbestimmter maximaler Druck in dem extrakorporalen Kreislauf erreicht ist/ wird.

Bevorzugt wird somit vermieden, dass beim Druckaufbau in dem extrakorporalen Kreislauf Priming-/ Spül-/ Dialysierflüssigkeit an der Dialysierflüssigkeitsseite entweichen kann. Weiterhin wird bevorzugt vermieden, dass Priming-/ Spül-/ Dialysierflüssigkeit oder Luft an der extrakorporalen Seite entweichen kann. Es ist ferner von Vorteil, wenn auf der Dialysierflüssigkeitsseite durch die Flusspumpe derselbe Druck (berechnet gemäß Gleichung 1) wie beim Primen/ Priming erzeugt wird.

Der Druckaufbau wird bevorzugt solange durchgeführt/ vorgenommen, bis ein vorbestimmter maximaler Druck in dem extrakorporalen Kreislauf erreicht bzw. gemessen wird. Zur Druckmessung in dem extrakorporalen Kreislauf kann ein (der zumindest eine) Drucksensor verwendet werden, beispielsweise der venöse Drucksensor oder der arterielle Drucksensor oder der Dialysatoreingangsdrucksensor. Der vorbestimmte maximale Druck ist insbesondere von dem Dialysator oder von den in dem extrakorporalen Kreislauf vorhandenen Komponenten abhängig. Beispielsweise sollten die Spezifikationen der verwendeten Dialysatoren eingehalten werden, insbesondere der maximale Transmembrandruck, welcher vom Dialysatortyp/ vom verwendeten Dialysator abhängig ist, und grundsätzlich in Datenblättern von Dialysatoren mit angegeben ist. Weiterhin kann der maximale Druck in Abhängigkeit der in dem extrakorporalen Kreislauf vorhandenen Komponenten bestimmt werden, beispielsweise derart, dass ein maximales Volumen einer vorgesehenen/ vorhandenen Ausgleichskammer/ Luftfalle zur Bestimmung des maximalen Drucks herangezogen wird. Besonders bevorzugt ist jedoch, dass der maximale Druck in dem extrakorporalen Kreislauf kleiner oder gleich dem maximalen Transmembrandruck ist. Dabei gilt grundsätzlich, dass der extrakorporale Kreislauf schneller gespült werden kann, das heißt, dass weniger Wiederholungen des Spülvorgangs erforderlich sind, je höher der maximale Transmembrandruck ist. Bevorzugt ist der maximale Druck/ Überdruck kleiner als 500 mmHg, besonders bevorzugt kleiner als 390 mmHg. Es sind jedoch offenbarungsgemäß auch höhere Drücke grundsätzlich möglich bzw. denkbar.

Ferner ist es von Vorteil, wenn die Steuereinheit eingerichtet ist, zum Steuern des Spülens, wenn der vorbestimmte maximale Druck in dem extrakorporalen Kreislauf vorliegt, das Dialysatoreinlassventil zu schließen, die zumindest eine Flusspumpe zu stoppen, das Entlüftungsventil geschlossen zu halten, und das Dialysatorauslassventil zu öffnen. Dadurch wird der Druck in dem Dialysierflüssigkeitskreislauf verringert bzw. entfernt, und der Druckunterschied zwischen dem extrakorporalen Kreislauf und dem Dialysierflüssigkeitskreislauf erzeugt. **In** anderen Worten ist der Druckunterschied dann bevorzugt als Überdruck in dem extrakorporalen Kreislauf gespeichert, und der vorbestimmte maximale Druck/ der Druckunterschied bzw. Überdruck wird in dem extrakorporalen Kreislauf dann schließlich über den Flüssigkeitstransfer/ Fluidfluss über die Membran des Dialysators hin zu dem Dialysierflüssigkeitskreislauf und zu dem (geöffneten) Dialysatorauslassventil abgebaut.

Es ist von Vorteil, wenn zumindest zwei Flusspumpen vorgesehen sind, wobei eine zweite Flusspumpe, insbesondere Flusspumpe-Ausgang, den Flüssigkeitstransfer über die Membran des Dialysators von dem extrakorporalen Kreislauf zu dem Dialysierflüssigkeitskreislauf unterstützt. Insbesondere wenn die zweite Flusspumpe/ Flusspumpe-Ausgang, welche stromabwärts des Dialysators vorgesehen ist, okklusiv/ verschließend arbeitet, ist es zwingend, dass die Flusspumpe-Ausgang den Flüssigkeitstransfer unterstützt. Ansonsten gilt, dass der Relaxationsvorgang von der zweiten Flusspumpe unterstützt werden kann, jedoch nicht unterstützt werden muss.

In vorteilhafter Weise ist die Steuereinheit eingerichtet, zum Steuern des Spülens: wenn der vorbestimmte maximale Druck in dem extrakorporalen Kreislauf abgebaut ist, das Dialysatorauslassventil zu schließen, gegebenenfalls die zweite Flusspumpe/ Flusspumpe-Ausgang zu stoppen, und den gesamten Spülvorgang erneut durchzuführen.

Zusammengefasst ist die Steuereinheit somit bevorzugt eingerichtet, zum Steuern des Spülens:

a) das Dialysatoreinlassventil zu öffnen oder geöffnet zu halten, das zumindest eine Entlüftungsventil zu schließen oder geschlossen zu halten, das Dialysatorauslassventil zu schließen oder geschlossen zu halten, und die zumindest eine Flusspumpe anzusteuern, damit diese die Flüssigkeit, insbesondere Priming-/ Spül-/ Dialysierflüssigkeit mit einem erforderlichen Druck in den Dialysator pumpt, und zwar bis ein vorbestimmter maximaler Druck in dem extrakorporalen Kreislauf erreicht ist;

b) wenn der vorbestimmte maximale Druck in dem extrakorporalen Kreislauf vorliegt, das Dialysatoreinlassventil zu schließen, die zumindest eine Flusspumpe zu stoppen, das Entlüftungsventil geschlossen zu halten, und das Dialysatorauslassventil zu öffnen, damit der vorbestimmte maximale Druck in dem extrakorporalen Kreislauf über den Flüssigkeitstransfer über die Membran des Dialysators hin zu dem geöffneten Dialysatorauslassventil abgebaut wird; und

c) wenn der vorbestimmte maximale Druck in dem extrakorporalen Kreislauf abgebaut ist, das Dialysatorauslassventil zu schließen, und den Spülvorgang beginnend bei Schritt a) erneut durchzuführen.

Bevorzugt werden die Schritte a) bis c) in chronologischer Reihenfolge von der Steuereinheit durchgeführt. Weiter bevorzugt werden die Schritte a) bis c) zyklisch wiederholt, damit der Spülvorgang gemäß den Schritten a) bis c) zum geeigneten Spülen des Dialysators und des extrakorporalen Kreislaufs mehrfach durchgeführt wird.

In vorteilhafter Weise ist ein Blutpumpenadapter/ ein Pumpsegment des Blutschlauchs des extrakorporalen Kreislaufs beim Primen und Spülen nicht in eine Blutpumpe der extrakorporalen Blutbehandlungsvorrichtung eingesetzt. Somit ist offenbarungsgemäß bevorzugt beim Primen und Spülen keine Peristaltikpumpe erforderlich und Pyrogene/ Reststoffe werden in vorteilhafter Weise nicht aufgelöst, wenn Flüssigkeit beim Primen und Spülen mit der Peristaltikpumpe gepumpt wird.

Zusammengefasst wird eine extrakorporale Blutbehandlungsvorrichtung mit einem extrakorporalen Kreislauf, einem Dialysator und einem Dialysierflüssigkeitskreislauf bereitgestellt, wobei die extrakorporale Blutbehandlungsvorrichtung zum Primen und Spülen derselben, vorbereitet bzw. eingerichtet ist und dazu ein arterieller Abschnitt und ein venöser Abschnitt des extrakorporalen Kreislaufs über eine Verbindervorrichtung verbunden sind, und wobei eine Steuereinheit der extrakorporalen Blutbehandlungsvorrichtung eingerichtet ist, das Primen derart zu steuern, dass eine Flüssigkeit von dem Dialysierflüssigkeitskreislauf über den Dialysator an den extrakorporalen Kreislauf geliefert wird, und das Spülen derart zu steuern, dass es über einen erzeugten Druckunterschied zwischen dem extrakorporalen Kreislauf und dem Dialysierflüssigkeitskreislauf erfolgt, zum Bewirken eines Flüssigkeitstransfers über den Dialysator, insbesondere von dem extrakorporalen Kreislauf zu dem Dialysierflüssigkeitskreislauf.

Weiterhin betrifft die vorliegende Offenbarung ein Verfahren zum Primen und Spülen eines extrakorporalen Kreislaufs und eines Dialysators einer extrakorporalen Blutbehandlungsvorrichtung, insbesondere wie voranstehend beschrieben, vor einem erstmaligen Verwenden des extrakorporalen Kreislaufs und des Dialysators mit den Schritten: Verbinden bzw. Kurzschließen eines arteriellen Abschnitts und eines venösen Abschnitts des extrakorporalen Kreislaufs der extrakorporalen Blutbehandlungsvorrichtung; Primen des extrakorporalen Kreislaufs und des Dialysators, indem eine Flüssigkeit von einem Dialysierflüssigkeitskreislauf der extrakorporalen Blutbehandlungsvorrichtung über eine Membran des Dialysators an den extrakorporalen Kreislauf geliefert wird; und ein gleichzeitig mit dem Primen oder unmittelbar nachfolgend auf das Primen durchzuführendes Spülen des extrakorporalen Kreislaufs und des Dialysators mit den Schritten: Erzeugen eines Druckunterschieds zwischen dem extrakorporalen Kreislauf und dem Dialysierflüssigkeitskreislauf; und Spülen des extrakorporalen Kreislaufs und des Dialysators über einen Flüssigkeitstransfer über die Membran des Dialysators, insbesondere von dem extrakorporalen Kreislauf zu dem Dialysierflüssigkeitskreislauf.

Bevorzugt enthält das Spülen den folgenden Schritt: Erzeugen des Druckunterschieds zwischen dem extrakorporalen Kreislauf und dem Dialysierflüssigkeitskreislauf derart, dass zunächst ein vorbestimmter maximaler Druck der Flüssigkeit, insbesondere Priming-/ Spül-/ Dialysierflüssigkeit, in dem Dialysierflüssigkeitskreislauf und dem extrakorporalen Kreislauf über ein in dem Dialysierflüssigkeitskreislauf vorgesehenes Druckerzeugungsmittel, insbesondere die zumindest eine Flusspumpe, erzeugt wird, und dann der vorbestimmte maximale Druck in dem Dialysierflüssigkeitskreislauf abgebaut wird.

**In** vorteilhafter Weise enthält das Primen folgende Schritte: Öffnen eines Dialysatoreinlassventils des Dialysierflüssigkeitskreislaufs und eines Entlüftungsventils des extrakorporalen Kreislaufs; Schließen eines Dialysatorauslassventils des Dialysierflüssigkeitskreislaufs; und Pumpen der Flüssigkeit, insbesondere Priming-/ Spül-/ Dialysierflüssigkeit, mit einem erforderlichen Druck in den Dialysator mittels einer in dem Dialysierflüssigkeitskreislauf vorgesehenen Flusspumpe.

Bevorzugt enthält das Spülen die folgenden Schritte: Öffnen oder Geöffnet Halten des Dialysatoreinlassventils, Schließen oder Geschlossen Halten des Entlüftungsventils, Schließen oder Geschlossen Halten des Dialysatorauslassventils, und Pumpen der Flüssigkeit, insbesondere Priming-/ Spül-/ Dialysierflüssigkeit mit einem erforderlichen Druck in den Dialysator, und zwar bis ein vorbestimmter maximaler Druck in dem extrakorporalen Kreislauf erreicht ist.

Es ist von Vorteil, wenn das Spülen ferner den folgenden Schritt enthält: wenn der vorbestimmte maximale Druck in dem extrakorporalen Kreislauf vorliegt, Schließen des Dialysatoreinlassventils, Stoppen der zumindest einen Flusspumpe, Geschlossen Halten des Entlüftungsventils, und Öffnen des Dialysatorauslassventils, und damit einhergehendes Abbauen des vorbestimmten maximalen Drucks in dem extrakorporalen Kreislauf über den Flüssigkeitstransfer über die Membran des Dialysators hin zu dem geöffneten Dialysatorauslassventil.

Weiter bevorzugt enthält das Spülen ferner den folgenden Schritt: Unterstützen des Flüssigkeitstransfers über die Membran des Dialysators von dem extrakorporalen Kreislauf zu dem Dialysierflüssigkeitskreislauf mittels einer Flusspumpe.

Zusammengefasst enthält das Spülen bevorzugt die folgenden Schritte: a) Öffnen oder Geöffnet Halten des Dialysatoreinlassventils, Schließen oder Geschlossen Halten des zumindest einen Entlüftungsventil, Schließen oder Geschlossen Halten des Dialysatorauslassventils, und Pumpen der Flüssigkeit, insbesondere Priming-/ Spül-/ Dialysierflüssigkeit, mit einem erforderlichen Druck in den Dialysator, und zwar bis ein vorbestimmter maximaler Druck in dem extrakorporalen Kreislauf erreicht ist; b) wenn der vorbestimmte maximale Druck in dem extrakorporalen Kreislauf vorliegt, Schließen des Dialysatoreinlassventils, Stoppen der zumindest einen Flusspumpe, Geschlossen Halten des Entlüftungsventil, und Öffnen des Dialysatorauslassventils, und damit einhergehendes Abbauen des vorbestimmten maximalen Drucks in dem extrakorporalen Kreislauf über den Flüssigkeitstransfer über die Membran des Dialysators hin zu dem geöffneten Dialysatorauslassventil; und c) wenn der vorbestimmte maximale Druck in dem extrakorporalen Kreislauf abgebaut ist, Schließen des Dialysatorauslassventils, und erneutes Durchführen des Spülvorgangs beginnend bei Schritt a).

Das Spülen/ der beschriebene Spülvorgang wird somit bevorzugt mehrfach, insbesondere sich zyklisch wiederholend durchgeführt.

Zusammengefasst stellt die vorliegende Offenbarung ein vollautomatisches Primen und Spülen bereit, wobei für das vollautomatische Primen und Spülen kein Substituat-Port, kein Waste-Port und keine zusätzlichen Einmal-/ Einwegartikel erforderlich sind.

Kurzbeschreibung der Figuren

Die Offenbarung wird nachfolgend mit Hilfe von Figuren weiter erläutert. Es zeigen:

Fig. 1 eine extrakorporale Blutbehandlungsvorrichtung gemäß der vorliegenden Offenbarung, anhand derer das offenbarungsgemäße automatische Primen und Spülen erläutert wird;

Fig. 2 ein Flussdiagramm des offenbarungsgemäßen automatischen Primens; und

Fig. 3 ein Flussdiagramm des offenbarungsgemäßen automatischen Spülens.

Figurenbeschreibung

Die Figuren sind lediglich schematischer Natur und dienen ausschließlich dem Verständnis der Offenbarung.

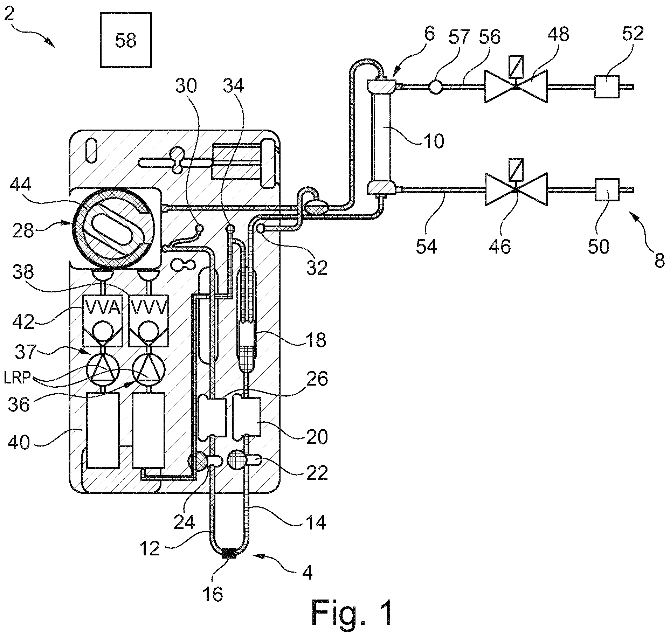

Fig. 1 zeigt eine extrakorporale Blutbehandlungsvorrichtung (Dialysemaschine) 2. Die extrakorporale Blutbehandlungsvorrichtung 2 enthält grundsätzlich einen extrakorporalen Kreislauf (A/V-Schlauchsystem) 4, einen Dialysator 6 und einen Dialysierflüssigkeitskreislauf 8. Dabei sind der extrakorporale Kreislauf 4 und der Dialysierflüssigkeitskreislauf 8 über eine in dem Dialysator 6 vorgesehene Membran 10 voneinander getrennt.

Der extrakorporale Kreislauf 4 enthält einen arteriellen Abschnitt 12, welcher sich stromaufwärts des Dialysators 6 befindet, und einen venösen Abschnitt 14, welcher sich stromabwärts des Dialysators 6 befindet.

Wie aus Fig. 1 ersichtlich ist, sind der arterielle Abschnitt 12 und der venöse Abschnitt 14 über einen Adapter 16, welcher ein Beispiel einer Verbindervorrichtung ist, verbunden bzw. kurzgeschlossen. In anderen Worten sind sowohl ein Ende des arteriellen Abschnitts 12 als auch ein Ende des venösen Abschnitts 14 in den Adapter 16 eingesteckt, damit der arterielle Abschnitt 12 und der venöse Abschnitt 14 fluidisch miteinander verbunden sind. Bevorzugt können in den Adapter 16 zwei Luer-Anschlüsse, welche bevorzugt an den Enden des arteriellen Abschnitts 12 und des venösen Abschnitts 14 vorgesehen sind, eingesteckt werden.

In dem venösen Abschnitt 14 des extrakorporalen Kreislaufs 4 sind stromabwärts des Dialysators 6 (das heißt ausgehend von dem Dialysator 6 in einer Richtung hin zu dem Adapter 16) eine venöse Expansionskammer bzw. Luftfalle 18, ein venöser Sicherheitsluftdetektor 20 und eine venöse Schlauchklemme 22 angeordnet bzw. vorgesehen.

In dem arteriellen Abschnitt 12 sind ausgehend von dem Adapter 16 in einer Richtung hin zu dem Dialysator 6 eine arterielle Schlauchklemme 24, ein arterieller Sicherheitsluftdetektor 26 und eine Blutpumpe 28 vorgesehen.

In dem arteriellen Abschnitt 12 kann ein arterieller Druck stromaufwärts bzw. vor der Blutpumpe 28 mit einem arteriellen Drucksensor 30 gemessen werden. Weiterhin kann ein Dialysatoreingangsdruck stromabwärts bzw. nach der Blutpumpe 28 und stromaufwärts bzw. vor dem Dialysator 6 (zwischen Dialysator 6 und Blutpumpe 28) über einen Dialysatoreingangsdrucksensor 32 gemessen werden. In dem venösen Abschnitt 14 kann ein venöser Druck an/ hinter der venösen Expansionskammer bzw. Luftfalle 18 über einen venösen Drucksensor 34 gemessen werden. Die in dem extrakorporalen Kreislauf 4 vorgesehenen Drucksensoren 30, 32, 34 können den Druck an den entsprechenden Stellen in dem extrakorporalen Kreislauf 4, an welchen diese angeordnet/ vorgesehen sind, messen/ abnehmen/ überwachen.

Wie aus Fig. 1 weiterhin hervorgeht, ist die venöse Expansionskammer bzw. Luftfalle 18 mit einer Pegel- oder Levelregulierungspumpe 36 verbunden. Die Pegel- oder Levelregulierungspumpe 36 dient einem Einstellen des Pegels der venösen Expansionskammer bzw. Luftfalle 18 und ist beispielsweise als ein Luftkompressor ausgebildet. Insbesondere kann die Pegel- oder Levelregulierungspumpe 36 Luft aus dem extrakorporalen Kreislauf 4 (insbesondere aus der venösen Expansionskammer bzw. Luftfalle 18) entfernen oder Luft in den extrakorporalen Kreislauf 4 (insbesondere in die venöse Expansionskammer bzw. Luftfalle 18) drücken, um so den Fluidpegel bzw. das Fluidlevel in der venösen Expansionskammer bzw. Luftfalle 18 einzustellen.

Hinter der Pegel- oder Levelregulierungspumpe 36 ist ein Entlüftungsventil, insbesondere ein venöses Entlüftungsventil 38 vorgesehen, über welches Luft aus dem extrakorporalen Kreislauf/ Schlauchsystem 4 entweichen kann. In Fig. 1 ist die venöse Expansionskammer/ Luftfalle 18 somit auch mit dem Entlüftungsventil 38 verbunden, so dass Luft aus der venösen Expansionskammer/ Luftfalle 18 bzw. aus dem extrakorporalen Kreislauf 4 grundsätzlich entweichen kann, wenn das Entlüftungsventil 38 geöffnet ist.

Wie aus Fig. 1 weiterhin hervorgeht, kann an einer Maschinenfront 40 der extrakorporalen Blutbehandlungsvorrichtung 2 eine weitere bzw. zweite Pegel- oder Levelregulierungspumpe 37 vorgesehen sein. Beispielsweise könnte die weitere bzw. zweite Pegel- oder Levelregulierungspumpe 37 mit einer (in Fig. 1 nicht vorgesehenen) arteriellen Expansionskammer bzw. Luftfalle verbunden werden, so dass grundsätzlich ein Fluidpegel bzw. ein Fluidlevel einer arteriellen Expansionskammer bzw. Luftfalle über die zweite Pegel- oder Levelregulierungspumpe 37 einstellbar wäre. Hinter dieser zweiten Pegel- oder Levelregulierungspumpe 37 ist in Fig. 1 ein weiteres bzw. zweites Entlüftungsventil, insbesondere ein arterielles Entlüftungsventil 42 angeordnet, über welches Luft aus einer arteriellen Expansionskammer/ Luftfalle und somit aus dem extrakorporalen Kreislauf 4 entweichen könnte.

Grundsätzlich ist es jedoch auch denkbar, dass auch wenn sowohl eine venöse Expansionskammer/ Luftfalle 18 als auch eine arterielle Expansionskammer/ Luftfalle vorgesehen sind, lediglich eine einzige Pegel- oder Levelregulierungspumpe vorgesehen ist, und diese - beispielsweise über ein zwischengeschaltetes Ventil - die Fluidpegel bzw. Fluidlevel von beiden Luftfallen einstellen kann. In diesem Fall könnte dann auch nur ein Entlüftungsventil hinter der einen Pegel- oder Levelregulierungspumpe vorgesehen sein.

Wie in Fig. 1 ersichtlich ist, ist der extrakorporale Kreislauf 4 (insbesondere ein Blutpumpenadapter 44 desselben) bereits in die Blutpumpe 28, welche bevorzugt als Rollenpumpe bzw. Peristaltikpumpe ausgebildet ist und eingerichtet ist, durch Quetschen eines Schlauchs ein Fluid/ eine Flüssigkeit zu fördern, eingesetzt. Gemäß der vorliegenden Offenbarung ist es jedoch bevorzugt, dass der Blutpumpenadapter 44 des extrakorporalen Kreislaufs 4 beim Primen und Spülen noch nicht in die Blutpumpe 28 eingesetzt ist, da die Blutpumpe 28 für das offenbarungsgemäße automatische Primen und Spülen nicht zwangsläufig benötigt wird. Wenn der Blutpumpenadapter 44 beim Primen und Spülen noch nicht in die Blutpumpe 28 eingesetzt ist, werden das Primen und Spülen in großem Maße vereinfacht. Insbesondere wird in diesem Fall der Blutpumpenadapter (Schlauch) 44 durch die (meist okkludierend arbeitende) Blutpumpe 28 nicht okkludiert bzw. gequetscht, und eine Ansteuerung der Blutpumpe 28 ist weder beim Primen noch beim Spülen vonnöten.

Der Dialysierflüssigkeitskreislauf 8 enthält ein Dialysatoreinlassventil 46, ein Dialysatorauslassventil 48, eine Flusspumpe-Eingang 50 und eine Flusspumpe-Ausgang 52. Grundsätzlich ist es jedoch ausreichend, wenn lediglich eine Flusspumpe, beispielsweise die Flusspumpe-Eingang 50, in dem Dialysierflüssigkeitskreislauf 8 vorgesehen ist. Das Dialysatoreinlassventil 46 und die Flusspumpe-Eingang 50 sind dabei an einem Dialysierflüssigkeitszufluss 54 stromaufwärts des Dialysators 6 vorgesehen/ angeordnet. Das Dialysatorauslassventil 48 und die Flusspumpe-Ausgang 52 sind an einem Dialysierflüssigkeitsabfluss 56 stromabwärts des Dialysators 6 vorgesehen/ angeordnet. Die Flusspumpe-Eingang 50 und die Flusspumpe-Ausgang 52 sind bevorzugt Zahnradpumpen. In dem Dialysierflüssigkeitsabfluss 56 ist in Fig. 1 ein Drucksensor 57 vorgesehen. Grundsätzlich kann offenbarungsgemäß jedoch der Drucksensor 57 auch in dem Dialysierflüssigkeitszufluss 54 angeordnet sein. Es ist auch denkbar, dass in dem Dialysierflüssigkeitskreislauf 8 mehrere Drucksensoren, beispielsweise zwei oder drei, vorgesehen sind. Es sollte jedoch in dem Dialysierflüssigkeitskreislauf 8 zumindest ein Drucksensor 57 vorgesehen sein, damit (zusammen mit dem in dem extrakorporalen Kreislauf 4 vorgesehenen Drucksensor/ den vorgesehenen Drucksensoren, beispielsweise arterieller Drucksensor 30, Dialysatoreingangsdrucksensor 32, venöser Drucksensor 34) der Transmembrandruck bestimmt werden kann.

Die extrakorporale Blutbehandlungsvorrichtung 2 weist weiterhin eine Steuereinheit 58 auf, welche bevorzugt als ein Prozessor, insbesondere als eine zentrale Rechen-/ bzw. Verarbeitungseinheit (CPU), ausgebildet ist. Die Steuereinheit 58 erhält Informationen von Sensoren, welche in der extrakorporalen Blutbehandlungsvorrichtung 2 vorgesehen sind. Lediglich beispielhaft sind dabei die in Fig. 1 gezeigten Sensoren zu nennen, also der arterielle Drucksensor 30, der Dialysatoreingangsdrucksensor 32, der venöse Drucksensor 34, oder der Drucksensor 57 in dem Dialysierflüssigkeitskreislauf 8. Auf der anderen Seite steuert bzw. betätigt die Steuereinheit 58 Aktoren, welche in der extrakorporalen Blutbehandlungsvorrichtung 2 vorgesehen sind. Lediglich beispielshaft sind dabei die in Fig. 1 gezeigten Ventile oder Pumpen, also insbesondere die Entlüftungsventile 38 und 42, das Dialysatoreinlassventil 46, das Dialysatorauslassventil 48, die Flusspumpe-Eingang 50, die Flusspumpe-Ausgang 52, die Blutpumpe 28, die Pegel- oder Levelregulierungspumpen 36, 37, etc. zu nennen.

Die Steuereinheit 58 steuert ein automatisches Primen. Insbesondere steuert sie das Primen derart, dass eine Flüssigkeit (Priming-/ Spül-/ bzw. Dialysierflüssigkeit) von dem Dialysierflüssigkeitskreislauf 8 über die Membran 10 des Dialysators 6 an den extrakorporalen Kreislauf 4 geliefert wird. Die Steuereinheit 58 öffnet dabei zunächst das Dialysatoreinlassventil 46 und das (venöse) Entlüftungsventil (VVV) 38. Wenn das (arterielle) Entlüftungsventil (VVA) 42 an den extrakorporalen Kreislauf 4, insbesondere an den arteriellen Abschnitt 12 des extrakorporalen Kreislaufs 4, angeschlossen wäre, würde die Steuereinheit 58 auch das (arterielle) Entlüftungsventil 42 öffnen. Ferner schließt die Steuereinheit 58 das Dialysatorauslassventil 48. In diesem Zustand steuert die Steuereinheit 58 die Flusspumpe-Eingang 50 an, damit diese die Flüssigkeit (Priming-/ Spül-/ bzw. Dialysierflüssigkeit) mit einem erforderlichen Druck in den Dialysator 6 pumpt.

Die Steuereinheit 58 ist bevorzugt eingerichtet, den erforderlichen Druck selbst zu berechnen, und stellt einen Parameter der Flusspumpe-Eingang, beispielsweise die Flussrate/ den Volumenstrom, entsprechend ein. Die für die Berechnung erforderlichen Informationen (siehe obige Gleichung 1), insbesondere die Volumina des Dialysators 6 und des extrakorporalen Kreislaufs 4, sowie den Ultrafiltrationskoeffizienten K$_{UF}$ erhält die Steuereinheit 58 beispielsweise über eine manuelle Eingabe (etwa über eine nicht gezeigte Benutzer-Schnittstelle) des verwendeten Schlauchsystems/ extrakorporalen Kreislaufs 4 und des verwendeten Dialysators 6. Alternativ können das verwendete Schlauchsystem 4 und der verwendete Dialysator 6 auch beispielsweise über ein nicht gezeigtes Lesegerät eingelesen werden, und die eingelesenen Informationen an die Steuereinheit 58 weitergegeben werden.

Bevorzugt wird die Flüssigkeit (Priming-/ Spül-/ bzw. Dialysierflüssigkeit) nicht nur über die Flusspumpe-Eingang 50 durch die Membran 10 des Dialysators 6 "gedrückt", sondern wird an einer Seite des extrakorporalen Kreislaufs 4 auch die Flüssigkeit durch Erzeugen eines Unterdrucks (negativen Drucks) durch die Membran 10 des Dialysators 6 "gezogen". Dieser Unterdruck/ negative Druck kann beispielsweise mittels der Pegel-oder Levelregulierungspumpe 36 erzeugt werden.

Beim Primen wird der Dialysator 6 und der extrakorporale Kreislauf 4 mit der Flüssigkeit (Priming-/ Spül-/ bzw. Dialysierflüssigkeit) befüllt. Eine Steuerung des Primens von der Steuereinheit 58 kann zeitgesteuert oder volumengesteuert erfolgen. Beispielsweise kann die Steuereinheit 58 im Vorhinein eine für das Primen erforderliche Zeit berechnen, und den Primingvorgang abbrechen, wenn diese berechnete erforderliche Zeit verstrichen ist. Alternativ kann das Luftvolumen, das den extrakorporalen Kreislauf 4 verlässt, auch mittels der Pegel- oder Levelregulierungspumpe 36 gemessen werden und die entsprechenden Informationen an die Steuereinheit 58 weitergegeben werden, und die Steuereinheit 58 beendet den Primingvorgang, wenn das Luftvolumen des Dialysators 6 und des extrakorporalen Kreislaufs 4 erreicht ist.

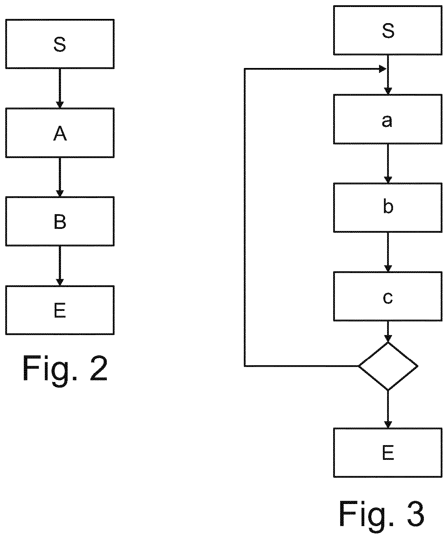

Das offenbarungemäße automatische Primen kann gemäß dem in Fig. 2 gezeigten Flussdiagramm somit in seiner einfachsten Umsetzung wie folgt zusammengefasst werden: Das automatische Primen wird bei "S" für "Start" gestartet. Bei A werden das Dialysatoreinlassventil 46 und das zumindest eine Entlüftungsventil 38 geöffnet oder bleiben geöffnet, und das Dialysatorauslassventil 48 wird geschlossen oder bleibt geschlossen. Bei B wird die Flusspumpe-Eingang 50 angesteuert, damit diese die Priming-/ Spül-/ Dialysierflüssigkeit mit einem erforderlichen Druck in den Dialysator 6 pumpt. Wenn ein Abbruchkriterium erfüllt ist (Zeitsteuerung oder Volumensteuerung), wird das automatische Primen bei "E" für "Ende" beendet.

Die Steuereinheit 58 steuert auch ein automatisches Spülen des Dialysators 6 und des extrakorporalen Kreislaufs 4. Bevorzugt findet das Spülen unmittelbar nachfolgend auf das Primen statt. Wenn das Primen abgeschlossen/ beendet ist, das heißt wenn der Dialysator 6 und der extrakorporale Kreislauf 4 mit der Flüssigkeit (Priming-/ Spül-/ bzw. Dialysierflüssigkeit) befüllt sind, schließt die Steuereinheit 58 das Dialysatoreinlassventil 46 und stoppt die Flusspumpe-Eingang 50. Das Entlüftungsventil 38 (bzw. die Entlüftungsventile 38 und 42) bleibt zunächst geöffnet und das Dialysatorauslassventil 48 bleibt geschlossen. Um das Spülen zu beginnen, öffnet die Steuereinheit 58 das Dialysatoreinlassventil 46 und schließt das Entlüftungsventil 38 (bzw. die Entlüftungsventile 38 und 42). Das Dialysatorauslassventil 48 bleibt geschlossen. In diesem Zustand steuert die Steuereinheit 58 die Flusspumpe-Eingang 50 an, damit diese die Flüssigkeit mit einem erforderlichen Druck in den Dialysator 6 pumpt. Die Flusspumpe-Eingang 50 erzeugt in anderen Worten einen Überdruck. Dies führt zunächst zu einem Druckanstieg in dem Dialysierflüssigkeitskreislauf 8, welcher sich durch den Flüssigkeitsübertritt über die Membran 10 auf den extrakorporalen Kreislauf 4 überträgt (Druckausgleich). Dieser Vorgang wird solange fortgesetzt, bis der Zieldruck (gewünschter Überdruck/ vorbestimmter maximaler Druck) in dem extrakorporalen Kreislauf 4 erreicht ist.

Der vorbestimmte maximale Druck in dem extrakorporalen Kreislauf 4 kann beispielsweise erreicht sein, wenn der maximale Transmembrandruck des verwendeten Dialysators 6 erreicht ist, oder wenn der maximale Pegelstand/ das maximale Fluidvolumen der venösen Expansionskammer/ Luftfalle 18 erreicht ist.

Wenn der vorbestimmte maximale Druck in dem extrakorporalen Kreislauf 4 vorliegt, schließt die Steuereinheit 58 das Dialysatoreinlassventil 46, stoppt die Flusspumpe-Eingang 50 und öffnet das Dialysatorauslassventil 48. Dadurch wird erreicht, dass sich der Druck auf der Dialysierflüssigkeitsseite/ in dem Dialysierflüssigkeitskreislauf 8 relaxieren kann. Es entsteht somit ein Druckunterschied zwischen dem extrakorporalen Kreislauf 4 und dem Dialysierflüssigkeitskreislauf 8. Dies hat zur Folge, dass der in dem extrakorporalen Kreislauf 4 gespeicherte Überdruck bzw. Druckunterschied über einen Fluidfluss über die Membran 10 des Dialysators 6 von dem extrakorporalen Kreislauf 4 zu dem Dialysierflüssigkeitskreislauf 8 hin zu dem geöffneten Dialysatorauslassventil 48 freigegeben bzw. abgebaut wird. Dieser Vorgang kann optional durch die Flusspumpe-Ausgang 52 unterstützt werden.

In anderen Worten wird offenbarungsgemäß das automatische Spülen realisiert, indem durch geeignete Ventil-/ und Pumpenbetätigungen zunächst ein Fluidfluss von dem Dialysierflüssigkeitskreislauf 8 über den Dialysator 6 zu dem extrakorporalen Kreislauf 4, und anschließend ein Fluidfluss von dem extrakorporalen Kreislauf 4 über den Dialysator 6 zu dem Dialysierflüssigkeitskreislauf 8 erfolgt. Der offenbarungsgemäße Fluidfluss wird erzeugt, indem zunächst ein maximaler Druck in dem extrakorporalen Kreislauf 4 erzeugt wird (Fluidfluss in die eine Richtung), dann der Druck in dem Dialysierflüssigkeitskreislauf 8 abgebaut wird, so dass der Druckunterschied zwischen dem extrakorporalen Kreislauf 4 und dem Dialysierflüssigkeitskreislauf 8 entsteht, und schließlich der in dem extrakorporalen Kreislauf 4 gespeicherte Druckunterschied/ Überdruck über einen Fluidfluss in die andere Richtung (zurück) abgebaut wird.

Nach einer erfolgten Druckrelaxation in dem extrakorporalen Kreislauf 4, schließt die Steuereinheit 58 das Dialysatorauslassventil 48 und der beschriebene Spülvorgang wird wiederholt (mehrmals), und zwar solange bis das erforderliche Spülvolumen erreicht ist.

Das offenbarungsgemäße automatische Spülen kann insgesamt gemäß dem in Fig. 3 gezeigten Flussdiagramm in seiner einfachsten Umsetzung wie folgt zusammengefasst werden: Das automatische Spülen wird bei "S" für "Start" gestartet. Bei a wird das Dialysatoreinlassventil 46 geöffnet oder bleibt geöffnet, das zumindest eine Entlüftungsventil 38 wird geschlossen oder bleibt geschlossen, das Dialysatorauslassventil 48 wird geschlossen oder bleibt geschlossen, und die Flusspumpe-Eingang 50 wird angesteuert, damit diese die Priming-/ Spül-/ Dialysierflüssigkeit mit einem erforderlichen Druck in den Dialysator 6 pumpt, und zwar bis ein vorbestimmter maximaler Druck in dem extrakorporalen Kreislauf 4 erreicht ist. Bei b wird, wenn der vorbestimmte maximale Druck in dem extrakorporalen Kreislauf 4 vorliegt, das Dialysatoreinlassventil 46 geschlossen, die Flusspumpe-Eingang 50 gestoppt, das Entlüftungsventil 38 wird geschlossen gehalten, und das Dialysatorauslassventil 48 wird geöffnet, damit der vorbestimmte maximale Druck in dem extrakorporalen Kreislauf 4 über den Flüssigkeitstransfer über die Membran 10 des Dialysators 6 hin zu dem geöffneten Dialysatorauslassventil 48 abgebaut wird. Bei c wird, wenn der vorbestimmte maximale Druck in dem extrakorporalen Kreislauf 4 abgebaut ist, das Dialysatorauslassventil 48 geschlossen. Anschließend wird geprüft, ob das erforderliche Spülvolumen bereits erreicht ist. Ist dies nicht der Fall, wird der Spülvorgang, das heißt die Schritte a, b und c erneut durchgeführt. Wenn das erforderliche Spülvolumen erreicht ist, wird das automatische Spülen bei "E" für "Ende" beendet.

Bezugszeichenliste

| | |
|---|---|
| 2 | extrakorporale Blutbehandlungsvorrichtung |
| 4 | extrakorporaler Kreislauf |
| 6 | Dialysator |
| 8 | Dialysierflüssigkeitskreislauf |
| 10 | Membran |
| 12 | arterieller Abschnitt |
| 14 | venöser Abschnitt |
| 16 | Adapter |
| 18 | venöse Expansionskammer/ Luftfalle |
| 20 | venöser Sicherheitsluftdetektor |
| 22 | venöse Schlauchklemme |
| 24 | arterielle Schlauchklemme |
| 26 | arterieller Sicherheitsluftdetektor |
| 28 | Blutpumpe |
| 30 | arterieller Drucksensor |
| 32 | Dialysatoreingangsdrucksensor |
| 34 | venöser Drucksensor |
| 36, 37 | Pegel- oder Levelregulierungspumpe |
| 38 | (venöses) Entlüftungsventil |
| 40 | Maschinenfront |
| 42 | (arterielles) Entlüftungsventil |
| 44 | Blutpumpenadapter/ Pumpsegment des Blutschlauchs |
| 46 | Dialysatoreinlassventil |
| 48 | Dialysatorauslassventil |
| 50 | Flusspumpe-Eingang |
| 52 | Flusspumpe-Ausgang |
| 54 | Dialysierflüssigkeitszufluss |
| 56 | Dialysierflüssigkeitsabfluss |
| 57 | Drucksensor |
| 58 | Steuereinheit |

Patentansprüche

1. Extrakorporale Blutbehandlungsvorrichtung (2) mit:

einem extrakorporalen Kreislauf (4) umfassend einen arteriellen Abschnitt (12) und einen venösen Abschnitt (14), einem Dialysator (6) und einem Dialysierflüssigkeitskreislauf (8), wobei

- der extrakorporale Kreislauf (4) und der Dialysierflüssigkeitskreislauf (8) über eine in dem Dialysator (6) vorgesehene Membran (10) voneinander getrennt sind;
- die extrakorporale Blutbehandlungsvorrichtung (2) zum Primen und Spülen des extrakorporalen Kreislaufs (4) und des Dialysators (6) vor einem erstmaligen Verwenden des extrakorporalen Kreislaufs (4) und des Dialysators (6) vorbereitet bzw. eingerichtet ist und dazu der arterielle Abschnitt (12) und der venöse Abschnitt (14) über eine Verbindervorrichtung (16) kurzgeschlossen bzw. verbunden sind;
- der Dialysierflüssigkeitskreislauf (8) ein Dialysatoreinlassventil (46), ein Dialysatorauslassventil (48) und zumindest eine Flusspumpe (50) aufweist;
- der extrakorporale Kreislauf (4) zumindest ein Entlüftungsventil (38) aufweist; und
- die extrakorporale Blutbehandlungsvorrichtung (2) ferner eine Steuereinheit (58) enthält, welche eingerichtet ist, das Primen derart zu steuern, dass eine Flüssigkeit, insbesondere Priming-/ Spül-/ Dialysierflüssigkeit, von dem Dialysierflüssigkeitskreislauf (8) über die Membran (10) des Dialysators (6) an den extrakorporalen Kreislauf (4) geliefert wird;

und wobei

die Steuereinheit (58) ferner eingerichtet ist, das gleichzeitig mit dem Primen oder unmittelbar nachfolgend auf das Primen durchzuführende Spülen der extrakorporalen Blutbehandlungsvorrichtung (2) derart zu steuern, dass zum Spülen ein Druckunterschied zwischen dem extrakorporalen Kreislauf (4) und dem Dialysierflüssigkeitskreislauf (8) erzeugt und ein Flüssigkeitstransfer über die Membran (10) des Dialysators (6), insbesondere von dem extrakorporalen Kreislauf (4) zu dem Dialysierflüssigkeitskreislauf (8), bewirkt wird,
**dadurch gekennzeichnet, dass**
die Steuereinheit (58) eingerichtet ist, zum Steuern des Primens

- das Dialysatoreinlassventil (46) und das zumindest eine Entlüftungsventil (38) zu öffnen oder geöffnet zu halten, und das Dialysatorauslassventil (48) zu schließen oder geschlossen zu halten; und
- die zumindest eine Flusspumpe (50), insbesondere Flusspumpe-Eingang, anzusteuern, damit diese die Flüssigkeit, insbesondere Priming-/ Spül-/ Dialysierflüssigkeit, mit einem erforderlichen Druck in den Dialysator (6) pumpt.

2. Extrakorporale Blutbehandlungsvorrichtung (2) nach Anspruch 1, **dadurch gekennzeichnet, dass** die Steuereinheit (58) eingerichtet ist, zum Steuern des Spülens den Druckunterschied zwischen dem extrakorporalen Kreislauf (4) und dem Dialysierflüssigkeitskreislauf (8) derart zu erzeugen, dass zunächst ein vorbestimmter maximaler Druck der Flüssigkeit, insbesondere Priming-/ Spül-/ Dialysierflüssigkeit, in dem Dialysierflüssigkeitskreislauf (8) und dem extrakorporalen Kreislauf (4) über ein in dem Dialysierflüssigkeitskreislauf (8) vorgesehenes Druckerzeugungsmittel, insbesondere die zumindest eine Flusspumpe (50), erzeugt wird, und dann der vorbestimmte maximale Druck in dem Dialysierflüssigkeitskreislauf (8) abgebaut wird.

3. Extrakorporale Blutbehandlungsvorrichtung (2) nach Anspruch 1 oder 2, **dadurch gekennzeichnet, dass** die Steuereinheit (58) eingerichtet ist, zum Steuern des Spülens:

- das Dialysatoreinlassventil (46) zu öffnen oder geöffnet zu halten,
- das zumindest eine Entlüftungsventil (38) zu schließen oder geschlossen zu halten,
- das Dialysatorauslassventil (48) zu schließen oder geschlossen zu halten, und
- die zumindest eine Flusspumpe (50) anzusteuern, damit diese die Flüssigkeit, insbesondere Priming-/ Spül-/ Dialysierflüssigkeit, mit einem erforderlichen Druck in den Dialysator pumpt, und zwar bis ein vorbestimmter maximaler Druck in dem extrakorporalen Kreislauf (4) erreicht ist.

4. Extrakorporale Blutbehandlungsvorrichtung (2) nach Anspruch 3, **dadurch gekennzeichnet, dass** die Steuereinheit (58) eingerichtet ist, zum Steuern des Spülens:

- wenn der vorbestimmte maximale Druck in dem extrakorporalen Kreislauf (4) vorliegt, das Dialysatoreinlassventil (46) zu schließen, die zumindest eine Flusspumpe (50) zu stoppen, das Entlüftungsventil (38) geschlossen zu halten, und das Dialysatorauslassventil (48) zu öffnen, damit der vorbestimmte maximale Druck in dem extrakorporalen Kreislauf (4) über den Flüssigkeitstransfer über die Membran (10) des Dialysators (6) hin zu dem geöffneten Dialysatorauslassventil (48) abgebaut wird.

5. Extrakorporale Blutbehandlungsvorrichtung (2) nach Anspruch 4, **dadurch gekennzeichnet, dass** zumindest zwei Flusspumpen (50, 52) vorgesehen sind, wobei eine zweite Flusspumpe (52), insbesondere Flusspumpe-Ausgang, den Flüssigkeitstransfer über die Membran (10) des Dialysators (6) von dem extrakorporalen Kreislauf (4) zu dem Dialysierflüssigkeitskreislauf (8) unterstützt.

6. Verfahren zum Primen und Spülen eines extrakorporalen Kreislaufs (4) und eines Dialysators (6) einer extrakorporalen Blutbehandlungsvorrichtung (2), insbesondere nach einem der vorhergehenden Ansprüche, vor einem erstmaligen Verwenden des extrakorporalen Kreislaufs (4) und des Dialysators (6), welches somit kein Anlegen und kein Behandeln eines Patienten umfasst, mit den Schritten:

Verbinden bzw. Kurzschließen eines arteriellen Abschnitts (12) und eines venösen Abschnitts (14) des extrakorporalen Kreislaufs (4) der extrakorporalen Blutbehandlungsvorrichtung (2); und
Primen des extrakorporalen Kreislaufs (4) und des Dialysators (6), indem eine Flüssigkeit von einem Dialysierflüssigkeitskreislauf (8) der extrakorporalen Blutbehandlungsvorrichtung (2) über eine Membran (10) des Dialysators (6) an den extrakorporalen Kreislauf (4) geliefert wird; wobei
gleichzeitig mit dem Primen oder unmittelbar nachfolgend auf das Primen ein Spülen des extrakorporalen Kreislaufs (4) und des Dialysators (6) durchgeführt wird mit den Schritten:

Erzeugen eines Druckunterschieds zwischen dem extrakorporalen Kreislauf (4) und dem Dialysierflüssigkeitskreislauf (8); und
Spülen des extrakorporalen Kreislaufs (4) und des Dialysators (6) über einen Flüssigkeitstransfer über die Membran (10) des Dialysators (6), insbesondere von dem extrakorporalen Kreislauf (4) zu dem Dialysierflüssigkeitskreislauf (8);
**dadurch gekennzeichnet, dass**
das Primen folgende Schritte enthält:

Öffnen oder Geöffnet Halten eines Dialysatoreinlassventils (46) des Dialysierflüssigkeitskreislaufs (8) und eines Entlüftungsventils (38) des extrakorporalen Kreislaufs (4);
Schließen oder Geschlossen Halten eines Dialysatorauslassventils (48) des Dialysierflüssigkeitskreislaufs (8); und
Pumpen der Flüssigkeit, insbesondere Priming-/ Spül-/ Dialysierflüssigkeit, mit einem erforderlichen Druck in den Dialysator (6) mittels einer in dem Dialysierflüssigkeitskreislauf (8) vorgesehenen Flusspumpe (50).

7. Verfahren zum Primen und Spülen einer extrakorporalen Blutbehandlungsvorrichtung (2) nach Anspruch 6, **dadurch gekennzeichnet, dass** das Spülen den folgenden Schritt enthält:
Erzeugen des Druckunterschieds zwischen dem extrakorporalen Kreislauf (4) und dem Dialysierflüssigkeitskreislauf (8) derart, dass zunächst ein vorbestimmter maximaler Druck der Flüssigkeit, insbesondere Priming-/ Spül-/ Dialysierflüssigkeit, in dem Dialysierflüssigkeitskreislauf (8) und dem extrakorporalen Kreislauf (4) über ein in dem Dialysierflüssigkeitskreislauf (8) vorgesehenes Druckerzeugungsmittel, insbesondere die zumindest eine Flusspumpe (50), erzeugt wird, und dann der vorbestimmte maximale Druck in dem Dialysierflüssigkeitskreislauf (8) abgebaut wird.

8. Verfahren zum Primen und Spülen einer extrakorporalen Blutbehandlungsvorrichtung (2) nach Anspruch 6 oder 7, **dadurch gekennzeichnet, dass** das Spülen ferner die folgenden Schritte enthält:

• Öffnen oder Geöffnet Halten des Dialysatoreinlassventils (46),
• Schließen oder Geschlossen Halten des Entlüftungsventils (38),
• Schließen oder Geschlossen Halten des Dialysatorauslassventils (48), und
• Pumpen der Flüssigkeit, insbesondere Priming-/ Spül-/ Dialysierflüssigkeit mit einem erforderlichen Druck in den Dialysator, und zwar bis ein vorbestimmter maximaler Druck in dem extrakorporalen Kreislauf (4) erreicht ist.

9. Verfahren zum Primen und Spülen einer extrakorporalen Blutbehandlungsvorrichtung (2) nach Anspruch 8, **dadurch gekennzeichnet, dass** das Spülen ferner den folgenden Schritt enthält:

• wenn der vorbestimmte maximale Druck in dem extrakorporalen Kreislauf (4) vorliegt, Schließen des Dialysatoreinlassventils (46), Stoppen der zumindest einen Flusspumpe (50), Geschlossen Halten des Entlüftungsventils (38), und Öffnen des Dialysatorauslassventil (48), und damit einhergehendes Abbauen des vorbestimmten maximalen Drucks in dem extrakorporalen Kreislauf (4) über den Flüssigkeitstransfer über die Membran (10) des Dialysators (6) hin zu dem geöffneten Dialysatorauslassventil (48).

10. Verfahren zum Primen und Spülen einer extrakorporalen Blutbehandlungsvorrichtung (2) nach Anspruch 9, **dadurch gekennzeichnet, dass** das Spülen ferner den folgenden Schritt enthält: Unterstützen des Flüssigkeitstransfers über die Membran (10) des Dialysators (6) von dem extrakorporalen Kreislauf (4) zu dem Dialysierflüssigkeitskreislauf (8) mittels einer Flusspumpe (52).

**Claims**

1. Extracorporeal blood treatment device (2) comprising:

   an extracorporeal circuit (4) comprising an arterial section (12) and a venous section (14), a dialyzer (6) and a dialyzing liquid circuit (8), wherein

   • the extracorporeal circuit (4) and the dialyzing liquid circuit (8) are separated from each other by a membrane (10) provided in the dialyzer (6);
   • the extracorporeal blood treatment device (2) is prepared or, respectively, configured for priming and rinsing of the extracorporeal circuit (4) and the dialyzer (6) prior to a first-time use of the extracorporeal circuit (4) and the dialyzer (6), and for this purpose, the arterial section (12) and the venous section (14) are short-circuited or, respectively, connected via a connector device (16);
   • the dialyzing liquid circuit (8) has a dialyzer inlet valve (46), a dialyzer outlet valve (48) and at least one flow pump (50);
   • the extracorporeal circuit (4) has at least one vent valve (38); and
   • the extracorporeal blood treatment device (2) further comprises a control unit (58), which is configured to control the priming such that a liquid, in particular priming/ rinsing/ dialyzing liquid, is supplied from the dialyzing liquid circuit (8) across the membrane (10) of the dialyzer (6) to the extracorporeal circuit (4);

   and wherein

   the control unit (58) is further adapted to control rinsing of the extracorporeal blood treatment device (2), to be performed simultaneously with the priming or immediately subsequent to the priming such that for rinsing, a pressure difference is created between the extracorporeal circuit (4) and the dialyzing liquid circuit (8), and a liquid transfer is effected across the membrane (10) of the dialyzer (6), in particular from the extracorporeal circuit (4) to the dialyzing liquid circuit (8),
   **characterized in that**
   the control unit (58) is adapted, for controlling of the priming,

   • to open or to keep open the dialyzer inlet valve (46) and the at least one vent valve (38), and to close or to keep closed the dialyzer outlet valve (48); and
   • to control the at least one flow pump (50), in particular flow pump-inlet, so that it pumps the liquid, in particular priming/ rinsing/ dialyzing liquid, into the dialyzer (6) at a required pressure.

2. The extracorporeal blood treatment device (2) according to claim 1, **characterized in that** the control unit (58) is adapted, for controlling the rinsing, to create the pressure difference between the extracorporeal circuit (4) and the dialyzing liquid circuit (8) such that initially, a predetermined maximum pressure of the liquid, in particular priming/ rinsing/ dialyzing liquid, is generated in the dialyzing liquid circuit (8) and the extracorporeal circuit (4) via pressure-generating means provided in the dialyzing liquid circuit (8), in particular the at least one flow pump (50), and then the predetermined maximum pressure is reduced in the dialyzing liquid circuit (8).

3. The extracorporeal blood treatment device (2) according to claim 1 or 2, **characterized in that** the control unit (58) is adapted, for controlling of the rinsing:

   • to open or to keep open the dialyzer inlet valve (46),
   • to close or to keep closed the at least one vent valve (38),
   • to close or to keep closed the dialyzer outlet valve (48), and
   • to control the at least one flow pump (50) so that it pumps the liquid, in particular priming/ rinsing/ dialyzing liquid, at a required pressure into the dialyzer, namely until a predetermined maximum pressure is reached in the extracorporeal circuit (4).

4. The extracorporeal blood treatment device (2) according to claim 3, **characterized in that** the control unit (58) is adapted, for controlling of the rinsing:

- if the predetermined maximum pressure is present in the extracorporeal circuit (4), to close the dialyzer inlet valve (46), to stop the at least one flow pump (50), to keep the vent valve (38) closed, and to open the dialyzer outlet valve (48), so that the predetermined maximum pressure in the extracorporeal circuit (4) is reduced via the liquid transfer across the membrane (10) of the dialyzer (6) towards the opened dialyzer outlet valve (48).

5. The extracorporeal blood treatment device (2) according to claim 4, **characterized in that** at least two flow pumps (50, 52) are provided, wherein a second flow pump (52), in particular a flow pump-outlet, supports the liquid transfer across the membrane (10) of the dialyzer (6) from the extracorporeal circuit (4) to the dialyzing liquid circuit (8).

6. A method for priming and rinsing of an extracorporeal circuit (4) and of a dialyzer (6) of an extracorporeal blood treatment device (2), in particular according to any of the preceding claims, prior to a first-time use of the extracorporeal circuit (4) and of the dialyzer (6), which thus does not include an application and treatment of a patient, comprising the steps of:

connecting or, respectively, short-circuiting of an arterial section (12) and a venous section (14) of the extracorporeal circuit (4) of the extracorporeal blood treatment device (2); and
priming of the extracorporeal circuit (4) and the dialyzer (6) by supplying a liquid from a dialyzing liquid circuit (8) of the extracorporeal blood treatment device (2) to the extracorporeal circuit (4) across a membrane (10) of the dialyzer (6), wherein
rinsing of the extracorporeal circuit (4) and the dialyzer (6) is performed simultaneously with the priming or immediately subsequent to the priming, comprising the steps of:

creating a pressure difference between the extracorporeal circuit (4) and the dialyzing liquid circuit (8); and
rinsing the extracorporeal circuit (4) and the dialyzer (6) via a liquid transfer across the membrane (10) of the dialyzer (6), in particular from the extracorporeal circuit (4) to the dialyzing liquid circuit (8),
**characterized in that**
the priming comprises the following steps:

opening or keeping open a dialyzer inlet valve (46) of the dialyzing liquid circuit (8) and a vent valve (38) of the extracorporeal circuit (4);
closing or keeping closed a dialyzer outlet valve (48) of the dialyzing liquid circuit (8); and
pumping the liquid, in particular priming/ rinsing/ dialyzing liquid, at a required pressure into the dialyzer (6) by means of a flow pump (50) provided in the dialyzing liquid circuit (8).

7. The method for priming and rinsing of an extracorporeal blood treatment device (2) according to claim 6, **characterized in that** the rinsing comprises the following step:
creating a pressure difference between the extracorporeal circuit (4) and the dialyzing liquid circuit (8) such that initially, a predetermined maximum pressure of the liquid, in particular priming/ rinsing/ dialyzing liquid, is created in the dialyzing liquid circuit (8) and the extracorporeal circuit (4) via pressure generating means, in particular via the at least one flow pump (50), provided in the dialyzing liquid circuit (8), and then the predetermined maximum pressure is reduced in the dialyzing liquid circuit (8).

8. The method for priming and rinsing of an extracorporeal blood treatment device (2) according to claim 6 or 7, **characterized in that** the rinsing further comprises the following steps:

- opening or keeping open the dialyzer inlet valve (46),
- closing or keeping closed the vent valve (38),
- closing or keeping closed the dialyzer outlet valve (48), and
- pumping of the liquid, in particular priming/ rinsing/ dialyzing liquid, at a required pressure into the dialyzer, namely until a predetermined maximum pressure is reached in the extracorporeal circuit (4).

9. The method for priming and rinsing of an extracorporeal blood treatment device (2) according to claim 8, **character-**

**ized in that** the rinsing further comprises the following step:

• if the predetermined maximum pressure is present in the extracorporeal circuit (4), closing of the dialyzer inlet valve (46), stopping of the at least one flow pump (50), keeping the vent valve (38) closed, and opening of the dialyzer outlet valve (48), and therewith associated, reducing of the predetermined maximum pressure in the extracorporeal circuit (4) via the liquid transfer across the membrane (10) of the dialyzer (6) towards the opened dialyzer outlet valve (48).

10. The method for priming and rinsing of an extracorporeal blood treatment device (2) according to claim 9, **characterized in that** the rinsing further comprises the following step: supporting of the liquid transfer across the membrane (10) of the dialyzer (6) from the extracorporeal circuit (4) to the dialyzing liquid circuit (8) by means of a flow pump (52).

**Revendications**

1. Dispositif de traitement extracorporel du sang (2) avec :

un circuit extracorporel (4) comprenant une section artérielle (12) et une section veineuse (14), un dialyseur (6) et un circuit de liquide de dialyse (8), dans lequel

- le circuit extracorporel (4) et le circuit de liquide de dialyse (8) sont séparés l'un de l'autre par le biais d'une membrane (10) prévue dans le dialyseur (6) ;
- le dispositif de traitement extracorporel du sang (2) destiné à l'amorçage et au rinçage du circuit extracorporel (4) et du dialyseur (6) est préparé ou configuré avant une première utilisation du circuit extracorporel (4) et du dialyseur (6) et à cet effet la section artérielle (12) et la section veineuse (14) sont court-circuitées ou reliées par le biais d'un dispositif connecteur (16) ;
- le circuit de liquide de dialyse (8) présente une vanne d'entrée de dialyseur (46), une vanne de sortie de dialyseur (48) et au moins une pompe à fluide (50) ;
- le circuit extracorporel (4) présente au moins une vanne de purge (38) ; et
- le dispositif de traitement extracorporel du sang (2) contient en outre un dispositif de commande (58) qui est configuré pour commander l'amorçage de telle sorte qu'un liquide, en particulier un liquide d'amorçage/de rinçage/de dialyse, soit amené au circuit extracorporel (4) du circuit de liquide de dialyse (8) par le biais de la membrane (10) du dialyseur (6) ;

et dans lequel
le dispositif de commande (58) est en outre configuré pour commander en même temps que l'amorçage ou immédiatement après l'amorçage le rinçage du dispositif extracorporel du sang (2) à exécuter de telle sorte que, pour le rinçage, une différence de pression soit générée entre le circuit extracorporel (4) et le circuit de liquide de dialyse (8) et un transfert de liquide soit effectué par le biais de la membrane (10) du dialyseur (6), en particulier du circuit extracorporel 4) au circuit de liquide de dialyse (8),
**caractérisé en ce que**
le dispositif de commande (58) est configuré pour la commande de l'amorçage

- pour ouvrir ou maintenir ouvertes la vanne d'entrée de dialyseur (46) et la au moins une vanne de purge (38) et fermer ou maintenir fermée la vanne de sortie de dialyseur (48) ; et
- commander la au moins une pompe à fluide (50), en particulier l'entrée de pompe à fluide, afin que celle-ci pompe le liquide dans le dialyseur (6), en particulier le liquide d'amorçage/de rinçage/de dialyse avec une pression nécessaire.

2. Dispositif de traitement extracorporel du sang (2) selon la revendication 1, **caractérisé en ce que** le dispositif de commande (58) est configuré pour générer la différence de pression entre le circuit extracorporel (4) et le circuit de liquide de dialyse (8) pour la commande du rinçage, de telle sorte qu'une pression maximale prédéterminée du liquide, en particulier du liquide d'amorçage/de rinçage/de dialyse, soit d'abord générée dans le circuit de liquide de dialyse (8) et le circuit extracorporel (4) par le biais d'un moyen de génération de pression prévu dans le circuit de liquide de dialyse (8), en particulier la au moins une pompe à fluide (50), et ensuite la pression maximale prédéterminée est réduite dans le circuit de liquide de dialyse (8).

3. Dispositif de traitement extracorporel du sang (2) selon la revendication 1 ou 2, **caractérisé en ce que** le dispositif de commande (58) est configuré pour la commande du rinçage :

- pour ouvrir ou maintenir l'ouverture de la vanne d'entrée de dialyseur (46),
- pour fermer ou maintenir la fermeture de la au moins une vanne de purge (38),
- pour fermer ou maintenir la fermeture de la vanne de sortie de dialyseur (48), et
- pour commander la au moins une pompe à fluide (50) afin que celle-ci pompe le liquide dans le dialyseur, en particulier le liquide d'amorce/de rinçage/de dialyse avec une pression nécessaire et jusqu'à ce qu'une pression maximale prédéterminée soit atteinte dans le circuit extracorporel (4).

4. Dispositif de traitement extracorporel du sang (2) selon la revendication 3, **caractérisé en ce que** le dispositif de commande (58) est configuré pour la commande du rinçage :

- lorsque la pression maximale prédéterminée dans le circuit extracorporel (4) est présente, pour fermer la vanne d'entrée de dialyseur (46), pour arrêter la au moins une pompe à fluide (50), pour maintenir fermée la vanne de purge (38) et pour ouvrir la vanne de sortie de dialyseur (48), afin que la pression maximale prédéterminée dans le circuit extracorporel (4) soit réduite par le biais du transfert de liquide via la membrane (10) du dialyseur (6) dans la vanne de sortie de dialyseur (48) ouverte.

5. Dispositif de traitement extracorporel du sang (2) selon la revendication 4, **caractérisé en ce qu'**au moins deux pompes à fluide (50, 52) sont prévues, dans lequel une seconde pompe à fluide (52), en particulier une sortie de pompe à fluide, aide le transfert de liquide via la membrane (10) du dialyseur (6) du circuit extracorporel (4) au circuit de liquide de dialyse (8).

6. Procédé destiné à l'amorçage et au rinçage d'un circuit extracorporel (4) et d'un dialyseur (6) d'un dispositif de traitement extracorporel du sang (2), en particulier selon l'une quelconque des revendications précédentes, avant une première utilisation du circuit extracorporel (4) et du dialyseur (6), procédé qui ne comprend ainsi aucun aménagement et aucun traitement d'un patient, avec les étapes suivantes :

liaison ou court-circuitage d'une section artérielle (12) et d'une section veineuse (14) du circuit extracorporel (4) du dispositif de traitement extracorporel du sang (2) ; et
amorçage du circuit extracorporel (4) et du dialyseur (6) en fournissant au circuit extracorporel (4) un liquide d'un circuit de liquide de dialyse (8) du dispositif de traitement extracorporel du sang (2) par le biais d'une membrane (10) du dialyseur (6) ; dans lequel
un rinçage du circuit extracorporel (4) et du dialyseur (6) est exécuté en même temps que l'amorçage ou immédiatement après l'amorçage avec les étapes suivantes :

génération d'une différence de pression entre le circuit extracorporel (4) et le circuit de liquide de dialyse (8) ; et
rinçage du circuit extracorporel (4) et du dialyseur (6) par le biais d'un transfert de liquide via la membrane (10) du dialyseur (6), en particulier du circuit extracorporel (4) au circuit de liquide de dialyse (8) ;
**caractérisé en ce que**
l'amorçage contient les étapes suivantes :

ouverture ou maintien de l'ouverture d'une vanne d'entrée de dialyseur (46) du circuit de liquide de dialyse (8) et d'une vanne de purge (38) du circuit extracorporel (4) ;
fermeture ou maintien de la fermeture d'une vanne de sortie de dialyseur (48) du circuit de liquide de dialyse (8) et
pompage du liquide, en particulier du liquide d'amorçage/de rinçage/de dialyse, avec une pression nécessaire dans le dialyseur (6) au moyen d'une pompe à fluide (50) prévue dans le circuit de liquide de dialyse (8).

7. Procédé destiné à l'amorçage et au rinçage d'un dispositif de traitement extracorporel du sang (2) selon la revendication 6, **caractérisé en ce que** le rinçage contient l'étape suivante :
génération de la différence de pression entre le circuit extracorporel (4) et le circuit de liquide de dialyse (8) de telle sorte qu'une pression maximale prédéterminée du liquide, en particulier du liquide d'amorçage/de rinçage/de dialyse, soit d'abord générée dans le circuit de liquide de dialyse (8) et le circuit extracorporel (4) par le biais d'un moyen de génération de pression prévu dans le circuit de liquide de dialyse (8), en particulier la au moins une pompe à fluide (50), et ensuite la pression maximale prédéterminée est réduite dans le circuit de liquide de dialyse (8).

8. Procédé destiné à l'amorçage et au rinçage d'un dispositif de traitement extracorporel du sang (2) selon la revendication 6 ou 7, **caractérisé en ce que** le rinçage contient en outre les étapes suivantes :

- ouverture ou maintien de l'ouverture de la vanne d'entrée de dialyseur (46),
- fermeture ou maintien de la fermeture de la vanne de purge (38),
- fermeture ou maintien de la fermeture de la vanne de sortie de dialyseur (48), et
- pompage du liquide, en particulier du liquide d'amorçage/de rinçage/de dialyse avec une pression nécessaire dans le dialyseur et jusqu'à ce qu'une pression maximale prédéterminée soit atteinte dans le circuit extracorporel (4).

9. Procédé destiné à l'amorçage et au rinçage d'un dispositif de traitement extracorporel du sang (2) selon la revendication 8, **caractérisé en ce que** le rinçage contient en outre l'étape suivante :

- lorsque la pression maximale prédéterminée est présente dans le circuit extracorporel (4), fermeture de la vanne d'entrée de dialyseur (46), arrêt de la au moins une pompe à fluide (50), maintien de la fermeture de la vanne de purge (38) et ouverture de la vanne de sortie de dialyseur (48) et par conséquent réduction de la pression maximale prédéterminée dans le circuit extracorporel (4) par le biais du transfert de fluide via la membrane (10) du dialyseur (6) dans la vanne de sortie de dialyseur (48) ouverte.

10. Procédé destiné à l'amorçage et au rinçage d'un dispositif de traitement extracorporel du sang (2) selon la revendication 9, **caractérisé en ce que** le rinçage contient en outre l'étape suivante : aide au transfert de liquide par le biais de la membrane (10) du dialyseur (6) du circuit extracorporel (4) au circuit de liquide de dialyse (8) au moyen d'une pompe à fluide (52).

Fig. 1

Fig. 2

Fig. 3

**IN DER BESCHREIBUNG AUFGEFÜHRTE DOKUMENTE**

**In der Beschreibung aufgeführte Patentdokumente**

- EP 3127564 B1
- EP 1457218 A1
- US 9579440 B2
- EP 2361643 B1
- WO 2013183599 A1